# EUROPEAN PATENT APPLICATION

(11) **EP 1 164 193 A1**
(43) Date of publication of application: **19.12.2001**
(21) Application number: 00202118.6
(22) Date of filing: 16.06.2000
(51) Int. Cl.: C12N 15/82, C07K 14/415, A01H 5/00

(54) **Plant-signalling ligand like proteins**

(71) Applicant: Plant Research International B.V., 6708 PB Wageningen (NL)
(72) Inventor: Liu, Chun-Ming, 6702 AS Wageningen (NL); Fiers, Martijn Adrianus, 6717 TJ Ede (NL); Cordewener, Johannes Hubertus Gerardus, 6708 MC Wageningen (NL); Joosen, Ronny Viktor Louis, 6871 VK Renkum (NL)
(74) Representative: Prins, Adrianus Willem

(57) **Abstract**

The invention relates to the field of plant growth and development, more in particular to the communication between plant cells influencing architectural or phenotypical characteristics such as their rate and pattern of division, orientation of elongation, organogenesis or differentiation patterns. The invention provides a method for modulating plant phenotype or architecture, such as by modulating or changing plant growth, it's development or defence responses, by modulating its rate or pattern of cell division, orientation of elongation, organogenesis or differentiation patterns, comprising providing a plant or plant material with recombinant ligand-like protein (LLP) or a functional fragment thereof, said protein or fragment at least comprising an LLP box as provided by the invention comprising an amino acid motif XRXXXXGXXXXHX.

### Annex to the application documents - subsequently filed sequences listing

SEQUENCE LISTING

<110> Stichting Dienst Landbouwkundig Onderzoek

<120> Plant-Signalling Ligand Like Proteins

<130> P53143EP00

<140> 00202118.6
<141> 2000-06-16

<160> 77

<170> PatentIn Ver. 2.1

<210> 1
<211> 531
<212> DNA
<213> Brassica napus

<220>
<223> Description of Combined DNA/RNA Molecule: Brassica napus

<220>
<221> CDS
<222> (31)..(330)

<400> 1
taacatgctt cctttatttt agtgcacccg tag ctt gta tat aaa att tta tcc 54
Leu Val Tyr Lys Ile Leu Ser
1               5

aca caa att cct ttt gct cct ttt atc aaa cgt cgc tat ttg tat cag 102
Thr Gln Ile Pro Phe Ala Pro Phe Ile Lys Arg Arg Tyr Leu Tyr Gln
        10                  15                  20

tct acc tat ctg ctt tca tat tgt atc tcc cgc agc caa aca aaa act 150
Ser Thr Tyr Leu Leu Ser Tyr Cys Ile Ser Arg Ser Gln Thr Lys Thr
25 30 35 40

ttt ttt ttg aca aag ata gaa cat gaa gat caa gag ttt gat att ggc 198
Phe Phe Leu Thr Lys Ile Glu His Glu Asp Gln Glu Phe Asp Ile Gly
45 50 55

ttc ctc ttt tct gat tct tgc ctt cat tca tca ctc aga atc agc ttc 246
Phe Leu Phe Ser Asp Ser Cys Leu His Ser Ser Leu Arg Ile Ser Phe
                60                  65                  70

att tcg gag ttt gct gat gaa gaa tgg att gta cga aga aga aga agc 294
Ile Ser Glu Phe Ala Asp Glu Glu Trp Ile Val Arg Arg Arg Arg Ser
            75                  80                  85

aaa aat tct att ggg cga ctc caa aga aac gat aac taattctaca 340
Lys Asn Ser Ile Gly Arg Leu Gln Arg Asn Asp Asn
90 95 100

gctttggagt ctaaacggat aattccgacg ggtccaaatc cacttcacaa caggtaactt 400

tgatcattta agaacaagat atgttgtgag tatgtctctt cctctgtttt tgttacaagt 460

atctatctta ctgtgtaatg taatggtgaa tgtataatac attttctaat taaattacct 520

tatttaaaaa a 531

<210> 2
<211> 99
<212> PRT
<213> Brassica napus
<223> Description of Combined DNA/RNA Molecule: Brassica napus

<400> 2
Leu Val Tyr Lys Ile Leu Ser Thr Gln Ile Pro Phe Ala Pro Phe Ile
1               5                   10                  15

Lys Arg Arg Tyr Leu Tyr Gln Ser Thr Tyr Leu Leu Ser Tyr Cys Ile
            20                  25                  30

Ser Arg Ser Gln Thr Lys Thr Phe Phe Leu Thr Lys Ile Glu His Glu
        35                  40                  45

Asp Gln Glu Phe Asp Ile Gly Phe Leu Phe Ser Asp Ser Cys Leu His
    50                  55                  60

Ser Ser Leu Arg Ile Ser Phe Ile Ser Glu Phe Ala Asp Glu Glu Trp
65                  70                  75                  80

Ile Val Arg Arg Arg Arg Ser Lys Asn Ser Ile Gly Arg Leu Gln Arg
                85                  90                  95

Asn Asp Asn

<210> 3
<211> 374
<212> DNA
<213> Brassica napus

<220>
<223> Description of Combined DNA/RNA Molecule: Brassica napus

<220>
<221> CDS

<222> (7)..(234)

<400> 3
caaaga tag aac atg aag atc aag agt ttg atg ttg gct tcc tct ttt    48
       Asn Met Lys Ile Lys Ser Leu Met Leu Ala Ser Ser Phe
         1               5                  10

ctg att ctt gcc ttc att cat cac tca gaa tca gct tca ttt cgg agt   96
Leu Ile Leu Ala Phe Ile His His Ser Glu Ser Ala Ser Phe Arg Ser
 15                  20                  25                  30

ttg ctg atg aag aat gta ttg tac gaa caa gaa gaa gca aaa att cta   144
Leu Leu Met Lys Asn Val Leu Tyr Glu Gln Glu Glu Ala Lys Ile Leu
                 35                  40                  45

ttg ggc gac tcc aaa gaa acg ata act aat tct aca gct ttg gag tct   192
Leu Gly Asp Ser Lys Glu Thr Ile Thr Asn Ser Thr Ala Leu Glu Ser
             50                  55                  60

aaa cgg ata att ccg acg ggt cca aat cca ctt cac aac agg          234
Lys Arg Ile Ile Pro Thr Gly Pro Asn Pro Leu His Asn Arg
         65                  70                  75

taaatttgat catttaagaa caagatatgt tgtgagtatg tctcttcctc ctctgttttt 294

gttacaagta tctatcttac tgtgtaatgt aatggtgaat gtataagaca ttttctaatt 354

aaattacctt atttaaaaaa                                             374


<210> 4
<211> 75
<212> PRT
<213> Brassica napus
<223> Description of Combined DNA/RNA Molecule: Brassica napus

<400> 4
Asn Met Lys Ile Lys Ser Leu Met Leu Ala Ser Ser Phe Leu Ile Leu
  1               5                  10                  15

Ala Phe Ile His His Ser Glu Ser Ala Ser Phe Arg Ser Leu Leu Met
             20                  25                  30

Lys Asn Val Leu Tyr Glu Gln Glu Glu Ala Lys Ile Leu Leu Gly Asp
         35                  40                  45

Ser Lys Glu Thr Ile Thr Asn Ser Thr Ala Leu Glu Ser Lys Arg Ile
     50                  55                  60

Ile Pro Thr Gly Pro Asn Pro Leu His Asn Arg
 65                  70                  75

<210> 5
<211> 107
<212> PRT
<213> Arabidopsis thaliana

<220>
<223> /note="LLP box genes"

<400> 5
Met Lys Thr Asn Arg Asn Arg Pro Ile Asn Ile Leu Ile Val Phe Phe
1               5                   10                  15

Leu Leu Thr Thr Ala Arg Ala Ala Thr Arg Asn Trp Thr Asn Arg Thr
            20                  25                  30

His Arg Thr Val Pro Lys Val Gln His Ala Tyr Tyr Ala Tyr Pro His
        35                  40                  45

Arg Ser Cys Glu Ser Phe Ser Arg Pro Tyr Ala Arg Ser Met Cys Ile
    50                  55                  60

Glu Leu Glu Arg Ile His Arg Ser Ser Arg Gln Pro Leu Phe Ser Pro
65                  70                  75                  80

Pro Pro Pro Pro Thr Glu Ile Asp Gln Arg Tyr Gly Val Glu Lys Arg
                85                  90                  95

Leu Val Pro Ser Gly Pro Asn Pro Leu His Asn
            100                 105

<210> 6
<211> 120
<212> PRT
<213> Arabidopsis thaliana

<220>
<223> /note="LLP box genes"

<400> 6
Met Thr Met Thr His Leu Asn Arg Leu Ile Leu Ile Ser Leu Leu Phe
1               5                   10                  15

Val Ser Leu Leu Leu Lys Ser Ser Thr Ala Ser Ser Thr Val Val Asp
            20                  25                  30

Glu Gly Asn Arg Thr Ser Arg Asn Phe Arg Tyr Arg Thr His Arg Phe
        35                  40                  45

Val Pro Arg Phe Asn His His Pro Tyr His Val Thr Pro His Arg Ser
    50                  55                  60

Cys Asp Ser Phe Ile Arg Pro Tyr Ala Arg Ser Met Cys Ile Glu Leu
65                  70                  75                  80

Gln Arg Ile His Arg Ser Ser Arg Lys Gln Pro Leu Leu Ser Pro Pro
                85                  90                  95

Pro Pro Glu Ile Asp Pro Arg Tyr Gly Val Asp Lys Arg Leu Val Pro
            100                 105                 110

Ser Gly Pro Asn Pro Leu His Asn
        115                 120

<210> 7
<211> 107
<212> PRT
<213> Arabidopsis thaliana

<220>
<223> /note="LLP box genes"

<400> 7
Met Ala Thr Thr Arg Val Ser His Val Leu Gly Phe Leu Leu Trp Ile
1               5                   10                  15

Ser Leu Leu Ile Phe Val Ser Ile Gly Leu Phe Gly Asn Phe Ser Ser
            20                  25                  30

Lys Pro Ile Asn Pro Phe Pro Ser Pro Val Ile Thr Leu Pro Ala Leu
        35                  40                  45

Tyr Tyr Arg Pro Gly Arg Arg Ala Leu Ala Val Lys Thr Phe Asp Phe
    50                  55                  60

Thr Pro Phe Leu Lys Asp Leu Arg Arg Ser Asn His Arg Lys Ala Leu
65                  70                  75                  80

Pro Ala Gly Gly Ser Glu Ile Asp Pro Arg Tyr Gly Val Glu Lys Arg
                85                  90                  95

Leu Val Pro Ser Gly Pro Asn Pro Leu His His
            100                 105

<210> 8
<211> 109
<212> PRT

<213> Arabidopsis thaliana

<220>
<223> /note="LLP box genes"

<400> 8
Met Ala Leu Lys Phe Ser Gln Ile Leu Phe Ile Val Leu Trp Leu Ser
1               5                   10                  15

Leu Phe Phe Leu Leu Leu His His Leu Tyr Ser Leu Asn Phe Arg Arg
            20                  25                  30

Leu Tyr Ser Leu Asn Ala Val Glu Pro Ser Leu Leu Lys Gln His Tyr
        35                  40                  45

Arg Ser Tyr Arg Leu Val Ser Arg Lys Val Leu Ser Asp Arg Phe Asp
    50                  55                  60

Phe Thr Pro Phe His Ser Arg Asp Asn Ser Arg His Asn His Arg Ser
65                  70                  75                  80

Gly Glu Gln Tyr Asp Gly Asp Glu Ile Asp Pro Arg Tyr Gly Val Glu
                85                  90                  95

Lys Arg Arg Val Pro Ser Gly Pro Asn Pro Leu His His
            100                 105

<210> 9
<211> 102
<212> PRT
<213> Arabidopsis thaliana

<220>
<223> /note="LLP box genes"

<400> 9
Gly Asp Cys Ser Arg Leu Ser Arg Ser Arg Arg Ala Tyr Thr Thr Ser
1               5                   10                  15

Thr Thr Gly Tyr Ala Ala Val Thr Phe Cys Gly Ile Phe Val Ser Ala
            20                  25                  30

Gln Tyr Ser Ile Ile Ser Ser Ala Leu Phe Ala Pro Asp His Tyr Pro
        35                  40                  45

Ser Val Pro Arg Lys Ala Gly His Ser His Glu Met Ala Ser Phe Gln
    50                  55                  60

Ala Pro Lys Ala Thr Val Pro Phe Thr Gly Gln His Arg Glu Glu Glu
65                  70                  75                  80

Asn Arg Asp Glu Val Tyr Gln Asp Asp Thr Arg Leu Val His Thr Gly
            85                  90                  95

Pro Asn Pro Leu His Asn
        100

<210> 10
<211> 103
<212> PRT
<213> Arabidopsis thaliana

<220>
<223> /note="LLP box genes"

<400> 10
Met Glu Ala Cys Ser Arg Lys Arg Arg Arg Arg Arg Ala Tyr Thr Thr
1               5                   10                  15

Ser Thr Thr Gly Tyr Ala Ala Val Phe Phe Cys Gly Ile Phe Val Phe
            20                  25                  30

Ala Gln Phe Gly Ile Ser Ser Ser Ala Leu Phe Ala Pro Asp His Tyr
        35                  40                  45

Pro Ser Leu Pro Arg Lys Ala Gly His Phe His Glu Met Ala Ser Phe
    50                  55                  60

Gln Ala Pro Lys Ala Thr Val Ser Phe Thr Gly Gln Arg Arg Glu Glu
65                  70                  75                  80

Glu Asn Arg Asp Glu Val Tyr Lys Asp Asp Lys Arg Leu Val His Thr
                85                  90                  95

Gly Pro Asn Pro Leu His Asn
            100

<210> 11
<211> 74
<212> PRT
<213> Arabidopsis thaliana

<220>
<223> /note="LLP box genes"

<400> 11
Met Lys Ile Lys Ser Leu Ile Leu Ala Ser Ser Phe Leu Ile Leu Ala
1               5                   10                  15

Phe Ile His His Ser Glu Ser Ala Ser Phe Arg Ser Leu Leu Met Lys
            20                  25                  30

Asn Gly Leu Tyr Glu Glu Glu Glu Ala Lys Ile Leu Leu Gly Asp Ser
        35                  40                  45

Lys Glu Thr Ile Thr Asn Ser Thr Ala Leu Glu Ser Lys Arg Ile Ile
    50                  55                  60

Pro Thr Gly Pro Asn Pro Leu His Asn Arg
65                  70

<210> 12
<211> 74
<212> PRT
<213> Arabidopsis thaliana

<220>
<223> /note="LLP box genes"

<400> 12
Met Lys Ile Lys Gly Leu Met Ile Leu Ala Ser Ser Leu Leu Ile Leu
1               5                   10                  15

Ala Phe Ile His Gln Ser Glu Ser Ala Ser Met Arg Ser Leu Leu Met
            20                  25                  30

Asn Asn Gly Ser Tyr Glu Glu Glu Glu Gln Val Leu Lys Tyr Asp Ser
        35                  40                  45

Met Gly Thr Ile Ala Asn Ser Ser Ala Leu Asp Ser Lys Arg Val Ile
    50                  55                  60

Pro Thr Gly Pro Asn Pro Leu His Asn Arg
65                  70

<210> 13
<211> 85
<212> PRT
<213> Arabidopsis thaliana

<220>
<223> /note="LLP box genes"

<400> 13
Met Gly Gly Asn Gly Ile Arg Ala Leu Val Gly Val Ile Ala Ser Leu
1               5                   10                  15

Gly Leu Ile Val Phe Leu Leu Val Gly Ile Leu Ala Asn Ser Ala Pro
        20                  25                  30

Ser Val Pro Ser Ser Glu Asn Val Lys Thr Leu Arg Phe Ser Gly Lys
        35                  40                  45

Asp Val Asn Leu Phe His Val Ser Lys Arg Lys Val Pro Asn Gly Pro
    50                  55                  60

Asp Pro Ile His Asn Arg Phe Leu Ser Leu Leu Ser Arg Ile Phe Asn
65                  70                  75                  80

Leu Leu Leu Leu Leu
                85

<210> 14
<211> 118
<212> PRT
<213> Arabidopsis thaliana

<220>
<223> /note="LLP box genes"

<400> 14
Met Arg Asn Asn His Ser Leu Arg Leu Gln Leu Trp Phe Arg Thr Leu
1               5                   10                  15

Phe Thr Val Gly Val Val Thr Leu Leu Met Ile Asp Ala Phe Val Leu
        20                  25                  30

Gln Asn Asn Lys Glu Asp Asp Lys Thr Lys Glu Ile Thr Thr Ala Val
        35                  40                  45

Asn Met Asn Asn Ser Asp Ala Lys Glu Ile Gln Gln Glu Leu Glu Asp
    50                  55                  60

Gly Ser Arg Asn Asp Asp Leu Ser Tyr Val Ala Ser Lys Arg Lys Val
65                  70                  75                  80

Pro Arg Gly Pro Asp Pro Ile His Asn Arg Phe Leu Leu Leu Ser Arg
                85                  90                  95

Phe Ile Leu Ser Leu Leu Thr Asn Pro Tyr Pro Tyr Leu His Ile Cys
            100                 105                 110

Val Leu Asp Val Ser Val
        115

<210> 15
<211> 106
<212> PRT
<213> Arabidopsis thaliana

<220>
<223> /note="LLP box genes"

<400> 15
Met Leu Ile Leu Ser Ser Arg Tyr Ala Met Lys Arg Asp Val Leu Ile
1               5                   10                  15

Ile Val Ile Phe Thr Val Leu Val Leu Ile Ile Ile Ser Arg Ser Ser
            20                  25                  30

Ser Ile Gln Ala Gly Arg Phe Met Thr Thr Gly Arg Asn Arg Asn Leu
        35                  40                  45

Ser Val Ala Arg Ser Leu Tyr Tyr Lys Asn His His Lys Val Val Ile
    50                  55                  60

Thr Glu Met Ser Asn Phe Asn Lys Val Arg Arg Arg Ser Ser Arg Phe
65                  70                  75                  80

Arg Arg Lys Thr Asp Gly Asp Glu Glu Glu Glu Glu Lys Arg Ser Ile
                85                  90                  95

Pro Thr Gly Pro Asn Pro Leu His Asn Lys
            100                 105

<210> 16
<211> 99
<212> PRT
<213> Arabidopsis thaliana

<220>
<223> /note="LLP box genes"

<400> 16
Met Thr Lys Gln Pro Lys Pro Cys Ser Phe Leu Phe His Ile Ser Leu
1               5                   10                  15

Leu Ser Ala Leu Phe Val Phe Leu Leu Ile Ser Phe Ala Phe Thr Thr
            20                  25                  30

Ser Tyr Lys Leu Lys Ser Gly Ile Asn Ser Leu Gly His Lys Arg Ile
        35                  40                  45

Leu Ala Ser Asn Phe Asp Phe Thr Pro Phe Leu Lys Asn Lys Asp Arg

50                55                60

Thr Gln Arg Gln Arg Gln Ser Pro Ser Leu Thr Val Lys Glu Asn Gly
65                70                75                80

Phe Trp Tyr Asn Asp Glu Glu Arg Val Val Pro Ser Gly Pro Asn Pro
                85                90                95

Leu His His

<210> 17
<211> 80
<212> PRT
<213> Arabidopsis thaliana

<220>
<223> /note="LLP box genes"

<400> 17
Met Lys Val Trp Ser Gln Arg Leu Ser Phe Leu Ile Val Met Ile Phe
1               5                   10                  15

Ile Leu Ala Gly Leu His Ser Ser Ser Ala Gly Arg Lys Leu Pro Ser
            20                  25                  30

Met Thr Thr Thr Glu Glu Phe Gln Arg Leu Ser Phe Asp Gly Lys Arg
        35                  40                  45

Ile Leu Ser Glu Val Thr Ala Asp Lys Lys Tyr Asp Arg Ile Tyr Gly
    50                  55                  60

Ala Ser Ala Arg Leu Val Pro Lys Gly Pro Asn Pro Leu His Asn Lys
65                  70                  75                  80

<210> 18
<211> 99
<212> PRT
<213> Arabidopsis thaliana

<220>
<223> /note="LLP box genes"

<400> 18
Met Thr His Val Leu Val Arg Arg Gln Gly Gln Gly Lys Lys Arg Arg
1               5                   10                  15

Trp Asp Val Asn Met Thr Met Cys Phe Phe Leu Phe Phe Phe Val Phe

            20                  25                  30

Tyr Val Ser Phe Gln Ile Val Leu Ser Ser Ser Ala Ser Val Gly Tyr
        35                  40                  45

Ser Arg Leu His Leu Val Ala Ser Pro Pro Pro Pro Pro Pro Arg Lys
    50                  55                  60

Ala Leu Arg Tyr Ser Thr Ala Pro Phe Arg Gly Pro Leu Ser Arg Asp
65                  70                  75                  80

Asp Ile Tyr Gly Asp Asp Lys Arg Val Val His Thr Gly Pro Asn Pro
                85                  90                  95

Leu His Asn

<210> 19
<211> 103
<212> PRT
<213> Arabidopsis thaliana

<220>
<223> /note="LLP box genes"

<400> 19
Met Gly Asn Tyr Tyr Ser Arg Arg Lys Ser Arg Lys His Ile Thr Thr
1               5                   10                  15

Val Ala Leu Ile Ile Leu Leu Leu Leu Leu Phe Leu Phe Leu Tyr Ala
            20                  25                  30

Lys Ala Ser Ser Ser Ser Pro Asn Ile His His His Ser Thr His Gly
        35                  40                  45

Ser Leu Lys Lys Ser Gly Asn Leu Asp Pro Lys Leu His Asp Leu Asp
    50                  55                  60

Ser Asn Ala Ala Ser Ser Arg Gly Ser Lys Tyr Thr Asn Tyr Glu Gly
65                  70                  75                  80

Gly Gly Glu Asp Val Phe Glu Asp Gly Lys Arg Arg Val Phe Thr Gly
                85                  90                  95

Pro Asn Pro Leu His Asn Arg
            100

<210> 20

<211> 83
<212> PRT
<213> Arabidopsis thaliana

<220>
<223> /note="LLP box genes"

<400> 20
Met Lys Asn Lys Asn Met Asn Pro Ser Arg Pro Arg Leu Leu Cys Leu
1               5                   10                  15

Ile Val Phe Leu Phe Leu Val Ile Val Leu Ser Lys Ala Ser Arg Ile
            20                  25                  30

His Val Glu Arg Arg Arg Phe Ser Ser Lys Pro Ser Gly Glu Asn Arg
        35                  40                  45

Glu Phe Leu Pro Ser Gln Pro Thr Phe Pro Val Val Asp Ala Gly Glu
    50                  55                  60

Ile Leu Pro Asp Lys Arg Lys Val Lys Thr Gly Ser Asn Pro Leu His
65                  70                  75                  80

Asn Lys Arg

<210> 21
<211> 75
<212> PRT
<213> Arabidopsis thaliana

<220>
<223> /note="LLP box genes"

<400> 21
Met Val Ile Leu Leu Ser Tyr Met Leu His Leu Phe Cys Val Tyr Ser
1               5                   10                  15

Arg Val Gly Ala Ile Arg Ile Phe Pro Glu Thr Pro Ala Ser Gly Lys
            20                  25                  30

Arg Gln Glu Glu Asp Leu Met Lys Lys Tyr Phe Gly Ala Gly Lys Phe
        35                  40                  45

Pro Pro Val Asp Ser Phe Val Gly Lys Gly Ile Ser Glu Ser Lys Arg
    50                  55                  60

Ile Val Pro Ser Cys Pro Asp Pro Leu His Asn
65                  70                  75

<210> 22
<211> 80
<212> PRT
<213> Arabidopsis thaliana

<220>
<223> /note="LLP box genes"

<400> 22
Met Ala Ser Phe Lys Leu Trp Val Cys Leu Ile Leu Leu Leu Leu Glu
1               5                   10                  15

Phe Ser Val His Gln Cys Arg Pro Leu Val Ala Glu Glu Ser Pro Ser
            20                  25                  30

Asp Ser Gly Asn Ile Arg Lys Ile Met Arg Glu Leu Leu Lys Arg Ser
        35                  40                  45

Glu Glu Leu Lys Val Arg Ser Lys Asp Gly Gln Thr Val Leu Gly Thr
    50                  55                  60

Leu Asp Ser Lys Arg Leu Ser Pro Gly Gly Pro Asp Pro Arg His His
65                  70                  75                  80



<210> 23
<211> 96
<212> PRT
<213> Arabidopsis thaliana

<220>
<223> /note="LLP box genes"

<400> 23
Met Asp Ser Lys Ser Phe Val Leu Leu Leu Leu Leu Phe Cys Phe Leu
1               5                   10                  15

Phe Leu His Asp Ala Ser Asp Leu Thr Gln Ala His Ala His Val Gln
            20                  25                  30

Gly Leu Ser Asn Arg Lys Met Met Met Met Lys Met Glu Ser Glu Trp
        35                  40                  45

Val Gly Ala Asn Gly Glu Ala Glu Lys Ala Lys Thr Lys Gly Leu Gly
    50                  55                  60

Leu His Glu Glu Leu Arg Thr Val Pro Ser Gly Pro Asp Pro Leu His
65                  70                  75                  80

His His Val Asn Pro Pro Arg Gln Pro Arg Asn Asn Phe Gln Leu Pro
                85                  90                  95

<210> 24
<211> 86
<212> PRT
<213> Arabidopsis thaliana

<220>
<223> /note="LLP box genes"

<400> 24
Met Lys Val Leu Lys Arg Asp Ser Met Leu Leu Leu Ile Thr Leu Tyr
1               5                   10                  15

Phe Leu Leu Thr Thr Ser Met Ala Arg Gln Asp Pro Phe Leu Val Gly
            20                  25                  30

Val Glu Lys Asp Val Val Pro Ala Gly Thr Asp Leu Lys Gln Asn Lys
        35                  40                  45

Ala Lys Pro His Leu Pro Asn Leu Phe Arg Thr Met Arg Arg Val Pro
    50                  55                  60

Thr Gly Pro Asn Pro Leu His His Ile Ser Pro Pro Gln Pro Gly Ser
65                  70                  75                  80

Leu Asn Tyr Ala Arg Asn
                85

<210> 25
<211> 97
<212> PRT
<213> Arabidopsis thaliana

<220>
<223> /note="C-terminal LLP box genes"

<400> 25
Met Lys Arg Asp Val Leu Ile Ile Val Ile Phe Thr Val Leu Val Leu
1               5                   10                  15

Ile Ile Ile Ser Arg Ser Ser Ser Ile Gln Ala Gly Arg Phe Met Thr
            20                  25                  30

Thr Gly Arg Asn Arg Asn Leu Ser Val Ala Arg Ser Leu Tyr Tyr Lys
        35                  40                  45

Asn His His Lys Val Val Ile Thr Glu Met Ser Asn Phe Asn Lys Val
50                  55                  60

Arg Arg Arg Ser Ser Arg Phe Arg Arg Lys Thr Asp Gly Asp Glu Glu
65                  70                  75                  80

Glu Glu Glu Lys Arg Ser Ile Pro Thr Gly Pro Asn Pro Leu His Asn
                85                  90                  95

Lys

<210> 26
<211> 79
<212> PRT
<213> Arabidopsis thaliana

<220>
<223> /note="C-terminal LLP box genes"

<400> 26
Met Thr Met Cys Phe Phe Leu Phe Phe Phe Val Phe Tyr Val Ser Phe
1               5                   10                  15

Gln Ile Val Leu Ser Ser Ser Ala Ser Val Gly Tyr Ser Arg Leu His
            20                  25                  30

Leu Val Ala Ser Pro Pro Pro Pro Pro Pro Arg Lys Ala Leu Arg Tyr
        35                  40                  45

Ser Thr Ala Pro Phe Arg Gly Pro Leu Ser Arg Asp Asp Ile Tyr Gly
    50                  55                  60

Asp Asp Lys Arg Val Val His Thr Gly Pro Asn Pro Leu His Asn
65                  70                  75

<210> 27
<211> 78
<212> PRT
<213> Arabidopsis thaliana

<220>
<223> /note="C-terminal LLP box genes"

<400> 27
Met Asn Pro Ser Arg Pro Arg Leu Leu Cys Leu Ile Val Phe Leu Phe
1               5                   10                  15

Leu Val Ile Val Leu Ser Lys Ala Ser Arg Ile His Val Glu Arg Arg
            20                  25                  30

Arg Phe Ser Ser Lys Pro Ser Gly Glu Asn Arg Glu Phe Leu Pro Ser
        35                  40                  45

Gln Pro Thr Phe Pro Val Val Asp Ala Gly Glu Ile Leu Pro Asp Lys
    50                  55                  60

Arg Lys Val Lys Thr Gly Ser Asn Pro Leu His Asn Lys Arg
65                  70                  75

<210> 28
<211> 36
<212> PRT
<213> Artificial Sequence

<220>
<223> Description of Artificial Sequence: example for blast

<400> 28
Glu Glu Ala Lys Ile Leu Leu Gly Asp Ser Lys Glu Thr Ile Thr Asn
1               5                   10                  15

Ser Thr Ala Leu Glu Ser Lys Arg Ile Ile Pro Thr Gly Pro Asn Pro
            20                  25                  30

Leu His Asn Arg
        35

<210> 29
<211> 1089
<212> DNA
<213> Brassica napus

<220>
<221> promoter
<222> (1)..(1089)
<223> /note="Promoter for BnLLP1"

<400> 29
acttgtgcgt taataatttg gaacactata agcggagaag acataaaatg gaatgccaaa 60
taggagtacc actgccatgt atgtttctgc attaatactg tgcccatact cacatttaat 120
tasgagtatg ctgattttrc gcatgagtta tcaaacgtga cagaacgtta tagagacatg 180
ttagaaaagc acgaagcctg aaaaactgca aaagtttaca acaaaaaaaa ctgataacta 240
cmattcttgg catttattgt cctttatttt tgtaaatagg tcttaaatac gtaataattt 300
tcttttcttt ttgacgaaga tatgtcgctt aattgtgtgc taatgccgtc gttcatgatc 360
gtctttcatg catatggagt aactgaacat gacactgatt caaaaaaaaa aattaatttc 420

ttacgaagtt ttgaataata gtttttttac cacattaagt acactttaat aaaaaaggaa 480
cacagtctac aaatagtttg agatgacatt gactattatc atctaacttt tattctgcat 540
aattgattta tcaaaaataa ttataattta tagtataaat gtagcctcta aatttagatt 600
tggcgatgat aaccatgtcc taaacttaca acggaccagt aagcggtcac aaatctatcg 660
gaaaatttcg tccctatagc agaatgtttg ggtttcctct ttatttatta ttgtattcaa 720
tgataactgg tcggtttact gttaccataa atgtgtccac aggctcactt aatgaggggt 780
gatgcttatg aatcttcaaa tcaaagacat aatgaagaac tttttcatag tgaaacgaga 840
taaagatcga ggaacatgca tgattccaca taattaccta attatttttc agctaaagta 900
tttcttttaa tatgtaacat gcttccttta tttttagtgc accgtagctt gtatataaaa 960
ttttatccac acaaaytcct tttgctcctt ttatcaaacg tcgctatttg tatcagtcta 1020
cctatttgct ttcatattgt atctcccgca gccaaacaaa aacttttttt ttgacaaaga 1080
tagaacatg 1089

<210> 30
<211> 1273
<212> DNA
<213> Arabidopsis thaliana

<220>
<221> promoter
<222> (1)..(1273)
<223> /note="Promoter + 5' UTR + coding region from AtLLP1"

<400> 30
gaaagtcaag aaactgagag aaagagagat ggtgtgaagt tgaaaatgag gaaaataatc 60
cttttggggg aaaaagaagt taatgcgttt gattgaaatt gaaagtggct agtaggagga 120
agtaagtaag catgggttac attacaattt caccttttca cttaccttta aacttcactt 180
ccaaaataat tacacttttc atgtgctttg tttcaaaggt ttctctgata ctctcttctt 240
catataatag aacccatata taatgtacta tatatatata tatatatata tatgaaaact 300
ctaattaagg tgttaaagtt aattgggaat gcggaagaac cagaatatat tcacatttca 360
tggtaaatta taagtttttc gtttattgtg aaagttctta tgggatggac atattatgat 420
catgtagtta tatagattag ttgcaaaata caaatatcaa tttgcatgat tcatgttttt 480
tcgaaatgtg cattaacatt cgcctctata caaagaatgg tgatgataga attgtcataa 540
aaagaagatg gaccgctaag aggtcacaaa tctattgcaa ttcgtcgtcc ataatgaatc 600
gtaaatgtat ttttcttaga cctcatcaat gggtagcaga atatttgggt tttattcatt 660
aagaaaaact atttattgta tacaattata acatgtcggc tttactgtta tcataaatgt 720
gtccacaggc cacttaatga ggctaaggct tatgaatctt caaatcaaag acataatgaa 780
gaaccttttt ttacctatcg aaacgagaaa aagtggaaca tgcatgattt ccacataatt 840
acctaattat tcttaagcta tttcatttag tcaaatgctt catttattta ttttatatgc 900
accgtatagc ttgtatataa atatatccat tctaattctt tttgcccttt tatcaaacgt 960
cgctatttat aacaatccag cttttgcttt cctccatatt atatccccat caaacaaaca 1020
aaaacaatat ttgaccaaaa atagacaaat gaagataaag ggtttgatga tattggcttc 1080
ttctctcctg attcttgcct tcattcatca gtcggaatca gcttcaatgc ggagtttgct 1140
gatgaataat ggatcatatg aagaagaaga acaagttcta aagtatgact ccatgggaac 1200
gatcgctaac tcttctgctt tagactcaaa acgggtaatt cccactggtc caaatccact 1260
ccacaacagg taa 1273

<210> 31
<211> 74
<212> PRT

<213> Arabidopsis thaliana

<220>
<223> /note="Coding region AtLLP1"

<400> 31
Met Lys Ile Lys Gly Leu Met Ile Leu Ala Ser Ser Leu Leu Ile Leu
1               5                   10                  15

Ala Phe Ile His Gln Ser Glu Ser Ala Ser Met Arg Ser Leu Leu Met
            20                  25                  30

Asn Asn Gly Ser Tyr Glu Glu Glu Glu Gln Val Leu Lys Tyr Asp Ser
        35                  40                  45

Met Gly Thr Ile Ala Asn Ser Ser Ala Leu Asp Ser Lys Arg Val Ile
    50                  55                  60

Pro Thr Gly Pro Asn Pro Leu His Asn Arg
65                  70

<210> 32
<211> 1300
<212> DNA
<213> Arabidopsis thaliana

<220>
<221> promoter
<222> (1)..(1300)
<223> /note="Promoter + 5' UTR + coding region from AtLLP11"

<400> 32
tctagagtgc aatactggca tggctttgct cacacaatca ctgtgagaat caatgctata 60
aggacactat cctgaccatg tgtaagattc taaatgatat aggagaaact gatatatagt 120
aatgaaattg tggggccaga tctgttcaaa ctttggcttt actatgttag aaggcaagct 180
tttctttccc accaaggcca aaaggaagtt tgcttgtgtc atggaaacaa actggctcaa 240
tcttccctag ctgtcaatca gatatagatt cttttttttt cacagtttag atatgtttct 300
taatgttttt gctttagttt atcgtttaaa ggttatgaag aacaagcata catttcttgc 360
tgctaattgg cttttggtag ggagggaata cttaacaagg aagaaaaatt gtaggctcta 420
gctccttttc acaggtttgg ttatcttact tgtgtattgt tacattctta attgattttg 480
ttattgttta aaggaaacat ccaagtgatt taagatagtg gtaaggaaag tagtaggctt 540
gctaatacac tcagtcacaa aaggtacact gtcctctgct tttaaaatgg cttaaaatat 600
atgtagttaa gggagcttgt gaggtcccca atggtttgct gcatgaaaat tcacaaatca 660
aagcaaagaa aaaggtactc aacgggccca tatgttacat aaagatatgt tttttcttaa 720
ctttcacttg ctcttgcctc ctctctctct ctctacgtgg acacctacgt atgtatattg 780
ttgctttgtg ttgcttcatc tttaaatagg gccaatcacg gatttgtctt ccaaggtata 840
taaaaacaac ctaaatgctt gttttttgct tcccatttcg ctttcccctt ttttgagcct 900
cttctgtcca aagatatctc tctctattta tgtgacagtc acttcaccaa catcatggat 960
gttctgctca gtttattctt gggtttggtt ggtcagttgt ttatgttaaa caggaagctg 1020
tagggacata ggtttcagta tgggtggaaa tggcattaga gctttggttg gagtgattgc 1080

atctttgggt ttgattgtgt ttcttcttgt cggtatctta gcaaactctg caccaagtgt 1140
tccatcatca gaaaatgtca agactttgcg gtttagtggt aaggatgtga atctgtttca 1200
tgtaagcaag cgaaaagttc ctaatggacc tgatcctatc cacaacaggt tcttatccct 1260
tctctctcga attttcaatc ttttactttt gcttctttga 1300

<210> 33
<211> 86
<212> PRT
<213> Arabidopsis thaliana

<220>
<223> /note="Coding region AtLLP11"

<400> 33
Met Gly Gly Asn Gly Ile Arg Ala Leu Val Gly Val Ile Ala Ser Leu
1               5                   10                  15

Gly Leu Ile Val Phe Leu Leu Val Gly Ile Leu Ala Asn Ser Ala Pro
            20                  25                  30

Ser Val Pro Ser Ser Glu Asn Val Lys Thr Leu Arg Phe Ser Gly Lys
        35                  40                  45

Asp Val Asn Leu Phe His Val Ser Lys Arg Lys Val Pro Asn Gly Pro
    50                  55                  60

Asp Pro Ile His Asn Arg Phe Leu Ser Leu Leu Ser Arg Ile Phe Asn
65                  70                  75                  80

Leu Leu Leu Leu Leu Leu
                85

<210> 34
<211> 2050
<212> DNA
<213> Arabidopsis thaliana

<220>
<221> promoter
<222> (1)..(2050)
<223> /note="Promoter + 5' UTR + coding region from AtLLP12"

<400> 34
ggtatcgaat tcccgcctaa aattttgatt ttagcaagtg atcgagaaag gacagagatc 60
gaagaaggtt cgagagagcg aaaaagcgag actagttagg gtttgagagt cttttgtgtc 120
tgatgaccaa tgagagagga attattctct gttttcttcc gttggcttct ctctcaatat 180
aatcaacaaa ttacagtcag ccttttttta ttattattca agggctccgc gtgggcttca 240
cgcgctcctt caacacggcc cacttaacca gacaaatcaa ggatcttcca ttagctaaac 300
cgtattcgaa ccgagtaatg aatctctctt taaccggtgg tactcttatc caaacaaaaa 360

tatccacgac tatttatatc cgatttagat tgttatctct ttctatattt tttatttact 420
tgagtgaaaa acatgaattg ttattctaaa aaaccattat aagaccgaaa attatcaaaa 480
cataaataat gtaagttttc agtgttttgg gttggtcgga caggtctaat ccgatcgaga 540
catgcctaac aatatcttct acaatttaag atgaaacata cgatgaatac atacgtgtat 600
atttgctgat ataacatcag agacaaagtg cttgacgaga acaaaatgga ataatggtat 660
ttttggggat ttgagtttgt atacctgctc tctactatgt aaatgatgat ttgtctgctg 720
taaaataaat gggaatataa tatataaaaa cgtgatgact gatgaggata tattgaggta 780
catatcacat atggtgtttc ttgacaaata ttggacgtaa gtctttgttt tttcttctat 840
aaactttcag cttgatttct taaaaacatt tgttgcacaa gttgtaaata cgtttatcga 900
aggtagaaga tgcatattgg agaatcatat ccatatatgt atcatgtatg taatttagtt 960
cttgtgatac tgttttcttt attctctttc ttaactcata ataaagtaaa attaaaaaaa 1020
aaaaagtaat cttagaaaga ctaaaaaacg agaagcaaat atagatatac aacatttata 1080
taaaatggag aggaagaaga ttcaagtggt aagtggattt tttcatgtgt gtgagtgagt 1140
gtaaatgtgg gtgaagaaaa agggagtgaa gacaacatca ttttccccaa agaaagctaa 1200
gtcttagaat catcactcca attccctttt tggccccttc ttctctcttt tctatttctt 1260
tctacttttg acccccttca cataattacc ttcctctata tgctttatta tcacaccctc 1320
attactctct aaaactaacc actataagta tattattaac cacactaatt actaccactt 1380
gacctagtaa tcccagctaa gacctttgga aatgattcaa cacacaaaaa aaactttcgt 1440
taaaaattaa tgatatatat atttacaaac ctctatctag tttctcttct ctttatttgt 1500
agaaagtggt ggagaataag aaagggagag caacaaactt ttagaggaag tcataggtta 1560
aaagccccat agctttttct tccatcactt tcattcctct cttctcattc ttacagactt 1620
tgagagagaa tttatttaag agatcttgat cagatctttt atacttgcaa aaatcgattc 1680
taaatatata atctatacct tatagagaaa ctcttcatat cttgaagatt cctaagagat 1740
gggaaattac tactctagaa gaaaatctag gaaacacatc actacagttg ccttgatcat 1800
ccttcttctt cttttgtttc tgtttcttta cgctaaagct tcatcgtctt ctcctaatat 1860
acatcatcac tcaactcatg gaagcttgaa gaaatctgga aatttggatc caaagcttca 1920
tgatcttgac tccaatgctg cgtcatcaag aggatcaaaa tatactaatt atgaaggtgg 1980
tggtgaagat gtttttgaag atggcaagag aagggtcttc acaggtccta atccattgca 2040
caatagataa 2050

<210> 35
<211> 103
<212> PRT
<213> Arabidopsis thaliana

<220>
<223> /note="Coding region AtLLP12"

<400> 35
Met Gly Asn Tyr Tyr Ser Arg Arg Lys Ser Arg Lys His Ile Thr Thr
1               5                   10                  15

Val Ala Leu Ile Ile Leu Leu Leu Leu Leu Phe Leu Phe Leu Tyr Ala
            20                  25                  30

Lys Ala Ser Ser Ser Ser Pro Asn Ile His His His Ser Thr His Gly
        35                  40                  45

Ser Leu Lys Lys Ser Gly Asn Leu Asp Pro Lys Leu His Asp Leu Asp
    50                  55                  60

Ser Asn Ala Ala Ser Ser Arg Gly Ser Lys Tyr Thr Asn Tyr Glu Gly
65                  70                  75                  80

Gly Gly Glu Asp Val Phe Glu Asp Gly Lys Arg Arg Val Phe Thr Gly
                85                  90                  95

Pro Asn Pro Leu His Asn Arg
            100

<210> 36
<211> 1717
<212> DNA
<213> Arabidopsis thaliana

<220>
<221> promoter
<222> (1)..(1717)
<223> /note="Promoter + 5' UTR + coding region from AtLLP5"

<400> 36
attcttttat cttcggacaa ccgatttcca aaaagtgata cgtttgaatg attttactct 60
ctttttcttt cattcaaacg tatcactttt tttttgttta gctatgttta attaatcaca 120
tactatatct tataatatcc acaattttat agttccttat atctgaaata ctacaaaagc 180
attttggaat cgcataataa taatgatgat gggatctttg cataattctg acacgaagag 240
gaagatgcga aagaaggcaa gaagaagaga cgaaaccaaa gagagaaaga aagaggaaca 3[?]
aaacaaacac aaaacggcaa agtgcagtta atgaaacgac accctttttc caaactcatc 360
tctaacatac aaacatccaa aggaacaatt ttgtgttgta ttgaacaaaa ccaaacgtaa 420
tcactatttg cttacgtcac actatcttca gattttctca agagacgctt acataataaa 480
atatgaagtt ttctttgttg gtatttgtaa taattatttt gtagttaaat aactatgaaa 540
tcttaattga caattccttt cacacaatgc tactagtttt aatatacagt ctatgctcat 600
agccatttac ggtctaatgt tttagacttt agtcatgtat gtcaaaaatg ataaacaaaa 660
attattgaaa ggaatggtgg cacggttttt gtttagcata agatccaatg atccacattc 720
aaacccatta aattcaaatt ttcttgtata tataactgta ccaagtcatt acatcaataa 780
tgttccaaag taaacctatt ctacatcatc tatataaagc taactccaaa acgacaacaa 840
tttgaaattc ataccaaaaa tgggtaagta cgttggtggg tggtgcattt attgattttt 900
gaaagttctg tttttcccgt acgtctaaag aatcaatata taaattgttt atatttgtat 960
atgtaatttt agatacagaa tctaattata aagcaatgta caatgtaata attcttgaca 1020
attccagaac aagagacaaa actattttaa attaattttt atatatgctc gtacagctta 1080
aataatatac tgtatataca cagttgtaca ccaataatag tactatacca ctatttatca 1140
taattagata tgtgaaaata tgtactagta atgaaacgaa aaaaaaacta aattaatcaa 1200
ttctctgatc ttgtcttgta tttatatata accattcacg tgggccatta aatttgcatg 1260
aaagccactc ttcttctcac aaaacaaaac ttcaatcttt tgctctctct ctctcaactt 1320
cagaaatagt catagaagat agatctaacc cagagaagac aagacagaaa gaagagatat 138[?]
aaaatcgatg attagggttt aatcaagaag acgactttat gttagggttt aggtttggga 1440
tatgactcat gctcgagaat ggagaagctc tttgactact acacttctaa tggtgatctt 1500
gctttcttat atgctccatc tcttctgtgt atattcacgg gtaggggcaa ttcggatatt 1560
tccagaaact ccggcttcgg gtaagagaca agaagaagat ctaatgaaga agtacttcgg 1620
cgccgggaaa tttccaccgg tggattcttt tgtcggtaaa gggatcagtg agagtaaaag 1680
aatagtacca agttgtccgg atcctttgca taactag                          1717

<210> 37
<211> 91
<212> PRT
<213> Arabidopsis thaliana

<220>
<223> /note="Coding region AtLLP5"

<400> 37
Met Thr His Ala Arg Glu Trp Arg Ser Ser Leu Thr Thr Thr Leu Leu
1               5                   10                  15

Met Val Ile Leu Leu Ser Tyr Met Leu His Leu Phe Cys Val Tyr Ser
            20                  25                  30

Arg Val Gly Ala Ile Arg Ile Phe Pro Glu Thr Pro Ala Ser Gly Lys
        35                  40                  45

Arg Gln Glu Glu Asp Leu Met Lys Lys Tyr Phe Gly Ala Gly Lys Phe
    50                  55                  60

Pro Pro Val Asp Ser Phe Val Gly Lys Gly Ile Ser Glu Ser Lys Arg
65                  70                  75                  80

Ile Val Pro Ser Cys Pro Asp Pro Leu His Asn
                85                  90

<210> 38
<211> 1552
<212> DNA
<213> Arabidopsis thaliana

<220>
<221> promoter
<222> (1)..(1552)
<223> /note="Promoter + 5' UTR + coding region from AtLLP2"

<400> 38
actactgtgt tcacacgtcg actataattt agacctattg atacgatatg aattattgca 60
atcatctcct gcaaatataa tagcatcgat tacacatctt tataatcaaa gatttttatt 120
attttacttt cataattcca aagatttttt cataattaat aagttgcaat catgcctcct 180
ttttcgttcc ttgaattcta agaaacctcg atgcattagt ttaattttct ttacacggtt 240
tattacacat tatgaatttt cttttgaata tcaattattg accactatag atttgattta 300
agtacgtatt attactattt tggagtaatt aatattcgct ttcatggtct ttaagttttt 360
taacgttatc tgacttgata tttttttttt gttggcttca aactaagact ctgattattt 420
ttatttagtt gtttgttctt aaccaaaaag agattatgtt tatctataat aaacgtgaat 480
tactatttaa acataaaaat tcaatgcaaa tttaaatttc ctttaaaaga atcaaaacta 540
gttcttaatg atttttttct aatactatac gaaatggtac ttaacacata gataaataaa 600
tgaattcact aatagagtaa tattgtacat ccatatttta ttattattaa taaatctgtt 660

ttcagaattg caaaatttct ttctaataaa aaagataaac gcaaatatac acatcggtct 720
aaaatcgaaa tctctatgct agagaggaga actaattaat ataaacatgt ttaggtacta 780
ggttcaaaaa tattatgaac ctatttaaga cttgcaagct cttggtaaaa agtaaaaaca 840
aataatatac cgatcataaa actgcaatct acgacagttt tgactaccgc caaagctaac 900
aaaatatcag ccagtaggca aaattttgac gcaaaaataa gctggacagt tttgtaaaat 960
ggtgtacata tgtcatatga taaagcatct tgcgttgaaa agacaaaatc caactctctc 1020
gcatctcttg agctttacat tttgtcaact atatcatata taaatataga tgagtccttt 1080
catactctct tctaaaccgt ccaacaaaac ctttttagtc ttttttccaa aaacatacaa 1140
aacataaaaa aaaaacaaga atttagtttt gtggcctatt gattagcaca cccatatgct 1200
tagaatttct tcatcatctt ccatggcctt gaaattttct caaattctct tcatagtttt 1260
atggctatct ctcttctttc tcctcctcca ccacttgtac tccctcaact tccgtcgtct 1320
ctactctcta aacgcggtgg agccatcatt gttgaagcaa cactaccgta gctatagatt 1380
agtgagcaga aaagttctct ccgacagatt cgacttcaca cctttccata gccgagataa 1440
cagccgacat aaccatcgtt ccggcgagca atacgacggt gatgagatag accctcgtta 1500
cggagtggag aaacgccgtg ttccctccgg acccaatccg ttgcaccatt ga 1552

<210> 39
<211> 109
<212> PRT
<213> Arabidopsis thaliana

<220>
<223> /note="Coding region AtLLP2"

<400> 39
Met Ala Leu Lys Phe Ser Gln Ile Leu Phe Ile Val Leu Trp Leu Ser
1               5                   10                  15

Leu Phe Phe Leu Leu Leu His His Leu Tyr Ser Leu Asn Phe Arg Arg
            20                  25                  30

Leu Tyr Ser Leu Asn Ala Val Glu Pro Ser Leu Leu Lys Gln His Tyr
        35                  40                  45

Arg Ser Tyr Arg Leu Val Ser Arg Lys Val Leu Ser Asp Arg Phe Asp
    50                  55                  60

Phe Thr Pro Phe His Ser Arg Asp Asn Ser Arg His Asn His Arg Ser
65                  70                  75                  80

Gly Glu Gln Tyr Asp Gly Asp Glu Ile Asp Pro Arg Tyr Gly Val Glu
                85                  90                  95

Lys Arg Arg Val Pro Ser Gly Pro Asn Pro Leu His His
            100                 105

<210> 40
<211> 1383
<212> DNA

<213> Arabidopsis thaliana

<220>
<221> promoter
<222> (1)..(1383)
<223> /note="Promoter + 5' UTR + coding region from AtLLP7"

<400> 40
ggagggacgg gtttttgaat caacattaat aaaaaagtaa aatataattg atccactgtt 60
tcaatacggg ttaaatattt aattattatt ttttaagacc actaatatta aacatatcaa 120
atcatcctaa tttagaaaag gttatataaa accaaaaatg ttatgtggta tgtataatgt 180
tactatatat aaaattaaac tataaaatat aaatgtatta gagaatgata caatttgtaa 240
aacttttata tgtaataaat aattttcaaa tttttaaaat tactacttta aaaataaatc 300
acgggatggg taaagaaatt acagaacgga ttttattttg gaattgagtt atatggtgga 360
tgtatttgaa tcaatattta taaaatttta aaatattatt aatatgctgt tttagtaagg 420
gttaaaactt cagtttttta acaattgtct catggattcg ttgtatagcg ttacttaata 480
acaattataa actgtaaaat ataaatattt tataaaaata aaatttgcaa gttttaatat 540
atattaactt taaaaaataa attataccgc agtataccgc gggttaaaat ctagtttata 600
tatatttcac ttaattttgt ctatgttcac tatacatatg taaactcaag agtcaagact 660
attcaattat atcatcattt cgcaaactta taataacaaa ttatataata tagtgataag 720
tgaagttttg cttactattt atcaaagatt tcatcttatt ttcaacaatt ctttcaattt 780
gtaatgttat cagtaaaaaa attgtagtat ataatctgct agttaataat acgaaaatat 840
ttgttattat tgtacgaaat tatcaattat gataattgtg tagtcatatc aatgtaatga 900
atgctgctga ccattctatt tcttctctcc ttattcccct ttgctcctcc ttacttttac 960
taaaattccc tcaactctta cgataagagc agagagaaga agaagaagaa gaaagtaatc 1020
gtgatttgag agagagattc aaacatactt ttttcgatat ctatgttaat tttatcttca 1080
cgatatgcaa tgaaacgaga tgttttaatc attgtaatct ttacagtgtt agtattaatc 1140
attatctccc gttcaagttc gatccaagct ggaagattca tgactacagg aagaaatcgt 1200
aatttgagcg ttgctcgatc actatactac aagaatcatc acaaggttgt gatcacggag 1260
atgtcaaatt ttaataaagt tagacgaaga tcgtctagat ttcggagaaa gacggacggc 1320
gacgaggaag aagaagagaa acgatctatc cccacaggtc caaatccttt gcacaacaag 1380
tag 1383

<210> 41
<211> 97
<212> PRT
<213> Arabidopsis thaliana

<220>
<223> /note="Coding region AtLLP7"

<400> 41
Met Lys Arg Asp Val Leu Ile Ile Val Ile Phe Thr Val Leu Val Leu
1               5                   10                  15

Ile Ile Ile Ser Arg Ser Ser Ser Ile Gln Ala Gly Arg Phe Met Thr
            20                  25                  30

Thr Gly Arg Asn Arg Asn Leu Ser Val Ala Arg Ser Leu Tyr Tyr Lys
        35                  40                  45

Asn His His Lys Val Val Ile Thr Glu Met Ser Asn Phe Asn Lys Val
50                  55                  60

Arg Arg Arg Ser Ser Arg Phe Arg Arg Lys Thr Asp Gly Asp Glu Glu
65                  70                  75                  80

Glu Glu Glu Lys Arg Ser Ile Pro Thr Gly Pro Asn Pro Leu His Asn
                85                  90                  95

Lys

<210> 42
<211> 13
<212> PRT
<213> Artificial Sequence

<220>
<223> Description of Artificial Sequence: LLP Boxmotif

<220>
<223> /note="X=variant"

<400> 42
Xaa Arg Xaa Xaa Xaa Xaa Gly Xaa Xaa Xaa Xaa His Xaa
1               5                   10

<210> 43
<211> 13
<212> PRT
<213> Artificial Sequence

<220>
<223> Description of Artificial Sequence: consensus
      sequence LLP boxmotif

<220>
<223> /note="X on position 4 is  V/I, X on position 5 is
      P/H, X on position 6 is S/T, X on position 8 is
      P/S, X on position 9 is N/D, X on position 10 is
      P/H, X on position 11 is L/I, X on position 13 is
      H/N"

<400> 43
Lys Arg Xaa Xaa Xaa Xaa Gly Xaa Xaa Xaa Xaa His Xaa
1               5                   10

<210> 44
<211> 13
<212> PRT
<213> Brassica napus

<220>
<223> /note="LLP boxmotif"

<400> 44
Lys Arg Ile Ile Pro Thr Gly Pro Asn Pro Leu His Asn
1               5                   10

<210> 45
<211> 13
<212> PRT
<213> Arabidopsis thaliana

<220>
<223> /note="LLP boxmotif"

<400> 45
Lys Arg Leu Val Pro Ser Gly Pro Asn Pro Leu His Asn
1               5                   10

<210> 46
<211> 13
<212> PRT
<213> Arabidopsis thaliana

<220>
<223> /note="LLP boxmotif"

<400> 46
Lys Arg Leu Val Pro Ser Gly Pro Asn Pro Leu His His
1               5                   10

<210> 47
<211> 13
<212> PRT
<213> Arabidopsis thaliana

<220>
<223> /note="LLP boxmotif"

<400> 47

Lys Arg Arg Val Pro Ser Gly Pro Asn Pro Leu His Asn
1               5                   10

<210> 48
<211> 13
<212> PRT
<213> Arabidopsis thaliana

<220>
<223> /note="LLP boxmotif"

<400> 48
Lys Arg Arg Val Pro Ser Gly Pro Asn Pro Leu His His
1               5                   10

<210> 49
<211> 13
<212> PRT
<213> Arabidopsis thaliana

<220>
<223> /note="LLP boxmotif"

<400> 49
Lys Arg Leu Val His Ser Gly Pro Asn Pro Leu His Asn
1               5                   10

<210> 50
<211> 13
<212> PRT
<213> Arabidopsis thaliana

<220>
<223> /note=LLP boxmotif"

<400> 50
Lys Arg Val Ile Pro Ser Gly Pro Asn Pro Leu His Asn
1               5                   10

<210> 51
<211> 13
<212> PRT
<213> Arabidopsis thaliana

<220>
<223> note="LLP boxmotif"

<400> 51
Lys Arg Lys Val Pro Ser Gly Pro Asn Pro Leu His Asn
1               5                   10

<210> 52
<211> 13
<212> PRT
<213> Arabidopsis thaliana

<220>
<223> /note="LLP boxmotif"

<400> 52
Lys Arg Ser Ile Pro Ser Gly Pro Asn Pro Leu His Asn
1               5                   10

<210> 53
<211> 13
<212> PRT
<213> Arabidopsis thaliana

<220>
<223> /note="LLP boxmotif"

<400> 53
Lys Arg Lys Val Pro Asn Gly Pro Asn Pro Leu His Asn
1               5                   10

<210> 54
<211> 13
<212> PRT
<213> Arabidopsis thaliana

<220>
<223> /note="LLP boxmotif"

<400> 54
Lys Arg Lys Val Pro Arg Gly Pro Asn Pro Leu His Asn
1               5                   10

<210> 55

<211> 13
<212> PRT
<213> Arabidopsis thaliana

<220>
<223> /note="LLP boxmotif"

<400> 55
Lys Arg Ser Ile Pro Thr Gly Pro Asn Pro Leu His Asn
1               5                   10

<210> 56
<211> 13
<212> PRT
<213> Arabidopsis thaliana

<220>
<223> /note="LLP boxmotif"

<400> 56
Glu Arg Leu Val Pro Ser Gly Pro Asn Pro Leu His Asn
1               5                   10

<210> 57
<211> 13
<212> PRT
<213> Arabidopsis thaliana

<220>
<223> /note="LLP boxmotif"

<400> 57
Glu Arg Leu Val Pro Ser Gly Pro Asn Pro Leu His His
1               5                   10

<210> 58
<211> 13
<212> PRT
<213> Arabidopsis thaliana

<220>
<223> /note="LLP boxmotif"

<400> 58
Ala Arg Leu Val Pro Ser Gly Pro Asn Pro Leu His Asn
1               5                   10

<210> 59
<211> 13
<212> PRT
<213> Arabidopsis thaliana

<220>
<223> /note="LLP boxmotif"

<400> 59
Ala Arg Leu Val Pro Lys Gly Pro Asn Pro Leu His Asn
1               5                   10

<210> 60
<211> 13
<212> PRT
<213> Arabidopsis thaliana

<220>
<223> /note="LLP boxmotif"

<400> 60
Lys Arg Val Val Pro Ser Gly Pro Asn Pro Leu His Asn
1               5                   10

<210> 61
<211> 13
<212> PRT
<213> Arabidopsis thaliana

<220>
<223> /note="LLP boxmotif"

<400> 61
Lys Arg Val Val His Thr Gly Pro Asn Pro Leu His Asn
1               5                   10

<210> 62
<211> 13
<212> PRT
<213> Arabidopsis thaliana

<220>
<223> /note="LLP boxmotif"

<400> 62
Lys Arg Arg Val Pro Ser Gly Pro Asn Pro Leu His Asn
1               5                   10

<210> 63
<211> 13
<212> PRT
<213> Arabidopsis thaliana

<220>
<223> /note="LLP boxmotif"

<400> 63
Lys Arg Arg Val Phe Ser Gly Pro Asn Pro Leu His Asn
1               5                   10

<210> 64
<211> 13
<212> PRT
<213> Arabidopsis thaliana

<220>
<223> /note="LLP boxmotif"

<400> 64
Lys Arg Lys Val Pro Lys Gly Pro Asn Pro Leu His Asn
1               5                   10

<210> 65
<211> 13
<212> PRT
<213> Arabidopsis thaliana

<220>
<223> /note="LLP boxmotif"

<400> 65
Lys Arg Lys Val Lys Ser Gly Pro Asn Ser Leu His Asn
1               5                   10

<210> 66
<211> 13
<212> PRT

<213> Arabidopsis thaliana

<220>
<223> /note="LLP boxmotif"

<400> 66
Lys Arg Leu Ser Pro Gly Gly Pro Asn Pro Leu His Asn
1               5                   10

<210> 67
<211> 13
<212> PRT
<213> Arabidopsis thaliana

<220>
<223> /note="LLP boxmotif"

<400> 67
Met Arg Leu Val Pro Ser Gly Pro Asn Pro Leu His Asn
1               5                   10

<210> 68
<211> 4
<212> PRT
<213> Artificial Sequence

<220>
<223> Description of Artificial Sequence: submotif

<400> 68
Asn Pro Leu His
1

<210> 69
<211> 4
<212> PRT
<213> Artificial Sequence

<220>
<223> Description of Artificial Sequence: submotif

<400> 69
Asp Pro Leu His
1

<210> 70
<211> 4
<212> PRT
<213> Artificial Sequence

<220>
<223> Description of Artificial Sequence: submotif

<400> 70
Asn Pro Arg His
1

<210> 71
<211> 4
<212> PRT
<213> Artificial Sequence

<220>
<223> Description of Artificial Sequence: submotif

<400> 71
Asp Pro Arg His
1

<210> 72
<211> 14
<212> DNA
<213> Artificial Sequence

<220>
<223> Description of Artificial Sequence: primer

<220>
<221> misc_feature
<222> (1)..(14)
<223> /note="Wherein M can be A, C or G and N can be any
    nucleotide"

<400> 72
tttttttttt ttmn                                    14

<210> 73
<211> 29
<212> DNA
<213> Artificial Sequence

<220>
<223> Description of Artificial Sequence: adapter primer AP1

<220>
<221> misc_feature
<222> (1)..(29)

<400> 73
ccattcttca tcagcaaact ccgaaatga                                29


<210> 74
<211> 28
<212> DNA
<213> Artificial Sequence

<220>
<223> Description of Artificial Sequence: adapter primer AP2

<220>
<221> misc_feature
<222> (1)..(28)

<400> 74
cagaaaagag gaagccaata tcaaactc                                28


<210> 75
<211> 31
<212> DNA
<213> Artificial Sequence

<220>
<223> Description of Artificial Sequence: P312-1

<220>
<221> misc_feature
<222> (1)..(31)

<400> 75
cgctctagag ttctatcttt gtcaaaaaaa a                             31


<210> 76
<211> 26
<212> DNA
<213> Artificial Sequence

<220>
<223> Description of Artificial Sequence: P312-2

<220>

<221> misc_feature
<222> (1)..(26)

<400> 76
atataagctt actatagggc acgcgt                                26

<210> 77
<211> 5
<212> DNA
<213> Artificial Sequence

<220>
<223> Description of Artificial Sequence: CAAT-box

<220>
<221> misc_feature
<222> (1)..(5)

<400> 77
ccaat                                                       5

## Description

The invention relates to the field of plant growth and development, more in particular to the communication between plant cells influencing architectural or phenotypical characteristics such as their rate and pattern of division, orientation of elongation, organogenesis or differentiation patterns in response to developmental or environmental stimuli .

The fusion of egg and sperm produces a zygote (also called fertilized egg). The single-cell zygote goes through a successive cell division and expansion process to generate a massive amount of cells that contribute to the body of a plant which can vary from a giant tree to a small grass, or from a potato to a peanut. Plant cell divisions are highly regulated, which give each plant or part thereof a specific shape or architecture.

There is no doubt that a precise developmental mechanism is present in plant cells to regulate its rate and pattern of division, orientation of elongation, organogenesis and differentiation. Such a developmental program is controlled genetically by genes in the nuclei and to a lesser extent in the chloroplast and mitochondria. During the last fifteen years, molecular genetic approaches have been used extensively to dissect such developmental pathways, especially in model organisms such as *Arabidopsis, Petunia,* maize and *Antirrinum.*

These studies have for example led to the identification of genes regulating flower development (Yanofsky et al, 1990; Mandel et al, 1992; Jufuku et al, 1994; Weigel et al, 1992), embryogenesis (Lotan et al,1998), meristem identity (Long et al, 1996), light (COP1) and hormone signal transduction (PIN, ETR, BRI, brassicasteroid, GAI, Peng), etc. The products of a large number of these genes turn out to be transcription factors which can bind to the promoter regions of downstream genes to initiate or suppress developmental pathways. Transcription factors include Myb, MADS, KNOTTED and AP2, etc. Mutation in one of these genes often leads to homeotic conversion from one organ to another. The expression of these different genes defines organ identity and the fate of differentiation of certain cell types.

Different from animal development, plant cells do not migrate during development and fate in a plant cell is less determined than the fate of an animal cell. Therefore, some perturbations that would cause abnormal growth and development in animals fail to affect normal plant morphogenesis. For example, over-expression of the cell cycle gene cyclAt increases the mass of the root but not the structure and morphology, and the *tangled* mutation in maize that failed to execute normal longitudinal cell divisions, is relatively normal in morphology. In plants, each cell needs to communicate and co-ordinate with its surrounding cells. Although plant cell division follows certain patterns with a traceable fate map, laser ablation experiments have revealed that when one or more cells are killed, those cells next to it are able to replace such cells, and even cells originating from a different layer which have different developmental origin have this capability (van den Berg et al, 1995).

Therefore, position itself is a very important signal for plant development (Hake and Char, 1997). Now the questions arises how position signals are accessed, how they are transferred between cells and how plant cells can sense such signals. Small signal molecules such as auxin and ethylene can diffuse through cells walls, while receptor kinases contain extracellular domains for ligand binding (Fletcher and Meyerowitz, 2000). Some proteins, such as transcription factors and viral movement proteins, can travel between cells through plasmodesmata (Lucas et al, 1995; Citovsky and Zambryski, 1991).
Another way of cell-cell communication is through receptor-like kinases. Receptor kinases:
There are four classes of receptor like kinases in Arabidopsis now known, based on their N-terminal extra-cellular domain sequences. Group representatives are:

The leucine-rich repeat (LRR) group, which is the largest group. LRRs occurs in numerous eukaryotic proteins and are thought to be involved in protein-protein interactions. LRR is also present in several mammalian receptors for protein/peptide messages including nerve growth factor receptor. This group includes ERECTA, CLV1, CLV2 and BRI1 (BRASSINOSTEROID-INSENSITIVE1). BRI1 is most likely the receptor of brassinosteroid. The expression pattern of currently known receptor kinases can be used to refine the function of LRRs.

The S-domain group, which have extra-cellular domains related to the S-locus glycoprotein of Brassica species involved in self-incompatibility response. Three S-domain RLK are found in arabidopsis, but they are not involved in self-incompatibility since they are expressed in inappropriate locations, and the species does not display self-incompatibility.

The lectin-like domain group, related to legume lectins. They may bind to oligosaccharides such as elicitors derived from the breakdown of cell walls of pathogen or plant during fungal infection.

EGF repeat receptor, represented in Arabidopsis by WAK1 and WAK4. Extracellular domain is related to mammalian epidermal growth factor.

Without ligand, the receptor-like kinases usually are present as a monomer in the membrane. The binding of a ligand to their extracellular domains leads to the formation of homo- or hetero-oligomers, usually dimers, to initiate a down-stream signal tranduction pathway by protein phosphorylation. Such a signal transduction pathway has been studied extensively in animals and yeast. Since the first plant protein kinase was reported in 1989 (Lawton, et al, 1989), more than 500 of them have been identified in plants and 175 in *Arabidopsis thaliana* alone (Hardie, 1999). Most of these protein kinases are involved in intracellular signal transduction (calcium-depedent protein kinases), stress response (leucine-rich repeat receptor kinases) and cell cycle regulation (cyclin-depedent kinases). Some protein kinases, for example, members of the two-component histidine/aspartate kinase family, are involved in hormone signal transduction, for instance, ethylene and cytokinin (Chang and Meyerowitz, 1995; Kakimoto, 1996). The recently identified ERECTA, BRI1, CLAVATA1,CLAVATA2 and HAESA are examples of receptor-like kinases which may be involved in cell-cell communication (Ku et al, 1996; Clark et al, 1997; Jeong et al, 1999; Jinn et al, 2000). Based on the outcome of genomic sequencing of Arabidopsis, it is expected that there are more than 100 receptor-like kinases in the Arabidopsis genome (Fletcher and Meyerowitz, 2000). Mutation of *CLAVATA1, CLAVATA2* and *CLAVATA3* showed almost identical phenotypes, enlarged central domain of meristems and increased floral organ numbers (Leyser and Furner, 1992). *CLAVATA3,* which was cloned recently, encodes a small predicted extracellular protein with no significant homology to any known plant and animal proteins (Fletcher et al., 1999). Based on phenotypic and biochemical analysis, CLAVATA1 and CLAVATA3 are believed to be different components of a signal transduction pathway , although direct proof for such interaction is not yet available (Clark et al, 1995; Trotochaud et al, 1999). Based on these results, it is very likely that CLAVATA3 is a ligand protein identified from higher plants, which interacts with the CLAVATA1 receptor kinase.

The invention provides a method for modulating plant phenotype or architecture, such as by affecting or changing plant growth, its development or its defence responses against external stimuli or disease, by modulating its rate or pattern of cell division, orientation of elongation, organogenesis or differentiation patterns, comprising providing a plant or plant material with recombinant ligand-like protein (LLP) or a functional fragment thereof, said protein or fragment at least comprising an LLP boxmotif as provided by the invention comprising an approximate amino acid motif XRXXXXGXXXXHX or (1)R(4)G(4)H(1). In particular, a preferred box comprises a consensus sequence showing at least 80% homology with a preferred consensus sequence K **R** X (V/I) (P/H) (S/T) **G** (P/S) (N/D) (P/H) (L/I) **H** (H/N) (bold amino acids typically are most conserved).

The invention herewith provides an isolated or recombinant ligand-like protein (LLP) or functional fragment thereof from a plant, for example a plant such as *Brassica napus* (LLP1Bn, otherwise known as DD3-12) or *Arabidopsis thaliana* (LLP1At), and its use to manipulate or influence plant architecture or modulate phenotype. LLP nucleic acid as provided herein in general encode ligands or functional fragments thereof that interact with receptor kinases which bring about the required phenotype response in plant tissues.

These phenotype responses also include alterations of cell fate, stress-mediated, hormone-mediated and disease-mediated responses. The invention thus provides a group of ligand-like proteins (LLPs) or functional fragments thereof with similar peptide structure and a conserved domain relatively close to their C-terminal, such as for example seen in LLP1, which are used to manipulate plant growth, development and defence response, and provides isolated and/or recombinant nucleic acid encoding said ligand-like proteins (LLP's) or functional fragments thereof.

Altered nucleic acid sequences of this invention include deletions, insertions, substitutions of different nucleotides resulting in the polynucleotides that encode the same or are functionally equivalent. Deliberate amino acid substitution may be made on the basis of similarity in polarity, charge, solubility, hydrophobicity, and/or the amphipathetic nature of the residues as long as the biological activity of the polypeptide is retained. Included in the scope of the present invention are alleles of the polypeptides. As used herein, an 'allele' or 'allelic sequence' is an alternative form of the polypeptides described above. A 'functional fragment' as defined herein may be an allelic variant. Alleles result from a mutation, eg a change in the nucleic acid sequence, and generally produce altered mRNA or polypeptide whose structure or function may or may not be altered. Any given polypeptide may have none, or more allelic forms. Common allelic changes that give rise to alleles are generally ascribed to natural deletions, additions or substitutions of amino acids. Each of these types of changes may occur alone, or in combination with the others, one or more times in a given sequence.

It is envisaged that the polynucleotide sequence of the present invention can be used as probes for the isolation of similar sequences from other genomes (e.g corn, rice, canola, soyabean, cotton etc). By using as a probe the gene sequence(s) of the present invention, it would be possible by those skilled in the art to obtain comparable gene sequences. One aspect of the invention is to provide for hybridisation or PCR probes which are capable of detecting polynucleotide sequences, including genomic sequence(s), encoding the polypeptides of the invention, or closely related molecules. The specificity of the probe [whether it is made from a highly specific region, eg 10 unique nucleotides in the 5' regulatory region, or the nucleic acid sequence of the LLPlbox motif or a less specific region e.g. in the 3' region], and the stringency of the hybridisation or amplification (maximal, high, intermediate, low) will determine whether the probe identifies only naturally occurring sequence(s) encoding the polypeptide, allele's or related sequences. Probes may also be used for the detection of related sequences and preferably contain at least 50% of any of the nucleotides from any one of the LLP gene encoding sequences according to the present invention.

The LLP nucleic acids or functional fragments thereof as provided herein can function in quite diverse biological pathways, for example in:
manipulating plant architecture, both shoots and roots, manipulating embryo-endosperm interactions, male sterility, flower timing and organ identity, meristem activity, apoptosis (eg. suspensor vs embryo), stress (biotic and abiotic) response, senescence, leaf and fruit dropping, nutrition uptake from roots. Furthermore, they can be used in "regeneration". "Regeneration" used as a general term for many possible applications of the LLP genes, such as competence, outgrowth, root formation, organogenesis, differentiation, vegetative development, shoot apical meristems, inflorescent meristem development, axillary bud formation and activation, or other processes where cell-cell communication or defining the boundaries of organs play a role.

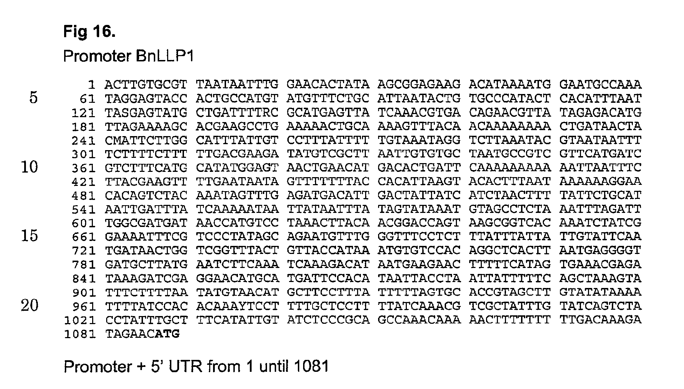

The invention also provides isolated and/or recombinant nucleic acids additionally comprising promoter sequences that are functionally linked or physically adjacent to the nucleic acid coding region of LLP1 and other LLPs or functional fragments thereof as mentioned herein, which act as regulating elements in plant cells for developmentally regulating tissue or cell-specific expression. The definition 'promoter' is intended as a nucleotide sequence sufficient to direct transcription. Also included are those promoter elements which are sufficient to render tissue-specific gene expression; such elements may be located in the 5' or 3' regions of the native gene. In the case of plant expression vectors, the expression of a sequence (s) of the invention may also be driven by a number of previously defined promoters, including inducible and developmentally regulated promoters. The invention further provides the use of the individual promoters of the polynucleotide sequence(s) of the present invention for this purpose [for example LLP1Bn promoter (Fig 16)].

The definition 'host cell' refers to a cell in which an foreign process is executed by bio-interaction, irrespective of the cell belongs to a unicellular, multicellular, a differentiated organism or to an artificial cell, cell culture or protoplast. The definition 'host cell' in the context of this invention is to also encompass the definition 'plant cell'.

'Plant cell' by definition is meant by any self-propagating cell bounded by a semi permeable membrane and containing one or more plastids. Such a cell often requires a cell wall if further propagation is required. 'Plant cell', as used herein, includes without limitation, seeds, suspension cultures, embryos, meristematic regions, callous tissues, protoplasts, leaves, roots, shoots, gametophytes, sporophytes, pollen and microspores.

More preferred, LLPs according to the invention also comprise a signal peptide at their N-terminals. The invention provides a method for selecting plant starting material or plants or their progenies for having a distinct LLP motif within one or more LLP genes. Such selection allows the detection of plants having a desired phenotype, by for example selecting plant (tissue) culture starting material, such as callus material or plants cells, having a desired LLP genotype. Selection can be preformed using nucleic acid detection methods known in the art, such as polymerase chain reaction (PCR) or by hybridisation, using LLP specific probes or primers herewith provided. Additionally, this invention also provides plants or plant material transformed with the nucleic acid sequences encoding the proteinaceous substances[protein, (poly)peptides and (post-translational) modifications thereof] as provided herein (LLP1 and other LLPs). Such plants have in general altered phenotypes. In short, the present invention, provides a new class of ligand-like proteins (LLPs) which are small proteins with a conserved LLP boxmotif relatively close to their C-terminals and a signal peptide at their N-terminals.

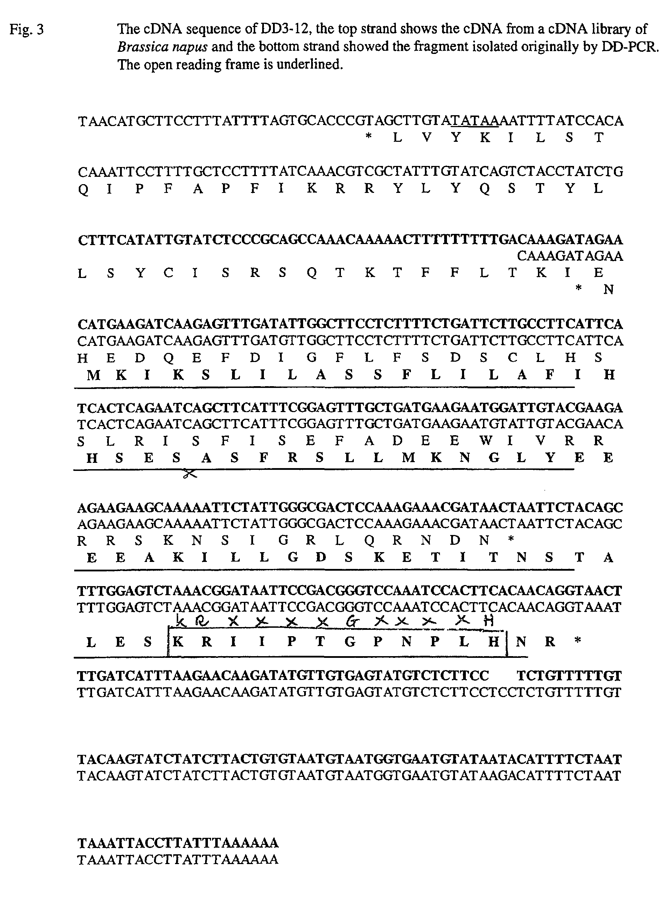

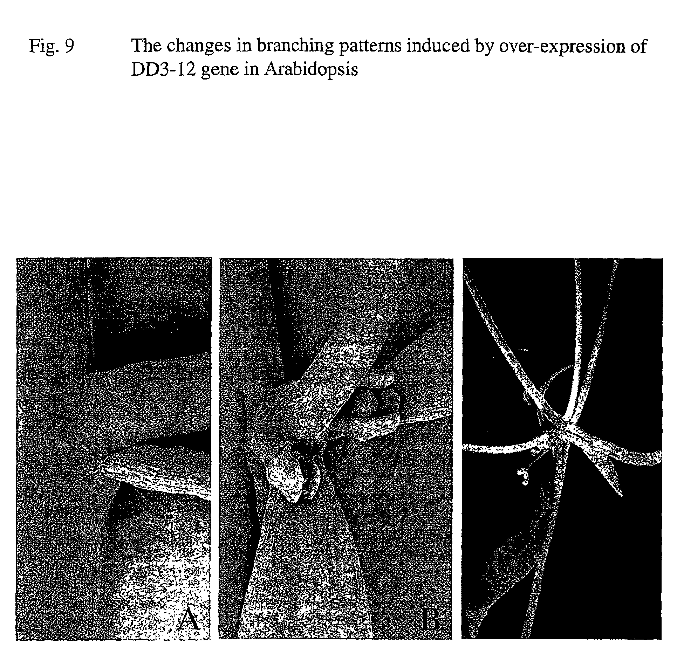

The invention furthermore provides a recombinant nucleic acid encoding a ligand-like protein or functional fragment thereof at least comprising an LLP boxmotif or peptide comprising an amino acid motif X**R**XXXX**G**XXXX**H**X, or a nucleic acid, such as anti-sense RNA, hybridising therewith. In one embodiment, the invention provides an LLP nucleic acid as shown in fig. 3. Over-expression of the LLP gene results in changes in plant architecture, such as male sterility or deviant root development (Figs 9-11). The invention also provides antisense LLP nucleic acid, primers or probes, be it of DNA, RNA or (peptide nucleic acid) PNA nature, hybridising with a nucleic acid as provided by the invention. Also provided is a nucleic acid according to the invention additionally provided with or comprising a promoter operably linked to a modified LLP nucleic acid. Such sequence can direct gene expression in axillary buds, floral organ primordium, stigma, and root-hair region and in the endosperm of mature and germinating seeds. Such a promoter is used to drive cell- or tissue-specific expression of a gene-of-interest. The invention furthermore provides a vector or host cell comprising a nucleic acid according to the invention, and a plant or plant material such as callus material or a plant cell provided or transformed with such a nucleic acid or vector.

The present invention is also related to the identification of a set of novel ligand-like proteins (LLP) that are structurally similar to LLP1. These proteins preferably have 50 or 60 or more amino acids, preferably have 75 or more amino acids, preferably have 85 or more amino acids, and preferably have no more than 250, even more preferred no more than 150 amino acids, and more preferably have no more than 120 amino acids; preferably they have a signal peptide at their N-terminals, said signal peptide preferably having a length of between 15 to 32 amino acids, as predicted by SignalP programme.

These proteinaceous substances have a conserved LLP boxmotif at their C-terminals, comprising amino acids X**R**XXXX**G**XXXX**H**X. Amino acids are herein given in the one-letter code, X stands for any naturally occurring amino acid. This LLP motif is preferably 55% or more, preferably 60% or more, more preferably 70% or more, more preferably 80% or more and most preferably 90% or more homologous to the LLP boxmotif or peptide as provided for *Brassica napus* (KRIIPTGPNPLHN; LLP boxmotif). Typical examples of an LLP boxmotif or peptide found in plants such as *Arabidopsis* are KRLVPSGPNPLHN, KRLVPSGPNPLHH, KRRVPSGPNPLHN, KRRVPSGPNPLHH, KRLVHSGPNPLHN, KRVIPSGPNPLHN, KRKVPSGPNPLHN, KRSIPSGPNPLHN, KRKVPNGPNPLHN, KRKVPRGPNPLHN, KRSIPTGPNPLHN, ERLVPSGPNPLHN, ERLVPSGPNPLHH, ARLVPSGPNPLHN, ARLVPKGPNPLHN, KRVVPSGPNPLHN, KRVVHTGPNPLHN, KRRVPSGPNPLHN, KRRVFSGPNPLHN, KRKVPKGPNPLHN, KRKVKSGPNSLHN, KRLSPGGPNPLHN, MRLVPSGPNPLHN, or variations of these wherein singular amino acids are replaced by like amino acids (e.g. basic by basic, bulky by bulky, or acid by acid) or wherein for example the NPLH sub-motif within an LLP boxmotif or peptide is replaced by DPLH, NPRH or DPRH. Proteins comprising an LLP boxmotif or peptide can easily be found (or mined in databases) by e.g. BLAST searches using an LLP boxmotif, performed on polypeptide sequences generated with recombinant techniques well known in the art. Variation should preferably not functionally affect the LLP boxmotif in the (1)R(4)G(4)H(1) position.

In general, the LLP [LLP1bn, LLP1at, and LLP homologs from other sources] have limited sequence homology to CLV3 proteins. CLV3 is a gene which functions as a regulator for the central zone of the apical meristem, possibly interacting with CLV1 receptor kinase although direct proof is still lacking (Fletcher et al, 1999). The LLP1 and other LLPs generally differ from CLV3 in at least one of three aspects: 1) very low homology, 21.1% identity in the overall protein sequences and homology in the LLP boxmotif is 54%; 2) no KR but instead LR is present; 3) preferred LLPs have the LLP boxmotif close to the end of the C-terminus of the protein, whereas CLV3 has a much longer C-terminal span. The length of the terminal peptide at the C-terminus of the LLP boxmotif should preferably be no more than 10 amino acids, more preferably no more than 5 amino acids and most preferably from 0 to 2 amino acids.

The invention provides also a recombinant nucleic acids comprising a promoter operably or functionally linked to LLP nucleic acids derived from different or heterologous plant species. Such sequences can direct gene expression in meristems, seeds or responding to abiotic and/or biotic stresses. Therefore, such a promoter is used to drive cell-, tissue-specific, stress-related expression of gene-of-interests.

The invention also provides a method for producing a plant having at least one or more cells transformed by LLPs nucleic acids, either by ectopic expression, misplaced-expression, over-expression, co-suppression or dominant-negative mutation. Down-regulation of these genes by anti-sense approaches can also be used within the scope. Over-expression of one or more of such genes in plants will lead to changes in plant growth, development and defence response. The invention is also related to the identification of receptor kinases which bind to ligand-like proteins like LLP1 and other LLPs. Such receptor kinases are generally membrane associated proteins with an extracellular domain and an intracellular domain, which can now be identified by reacting with a ligand-like protein or functional fragment thereof as provided herewith.

### Detailed description

### Example 1: Cloning of LLP1Bn gene (DD3-12) from Brassica napus L

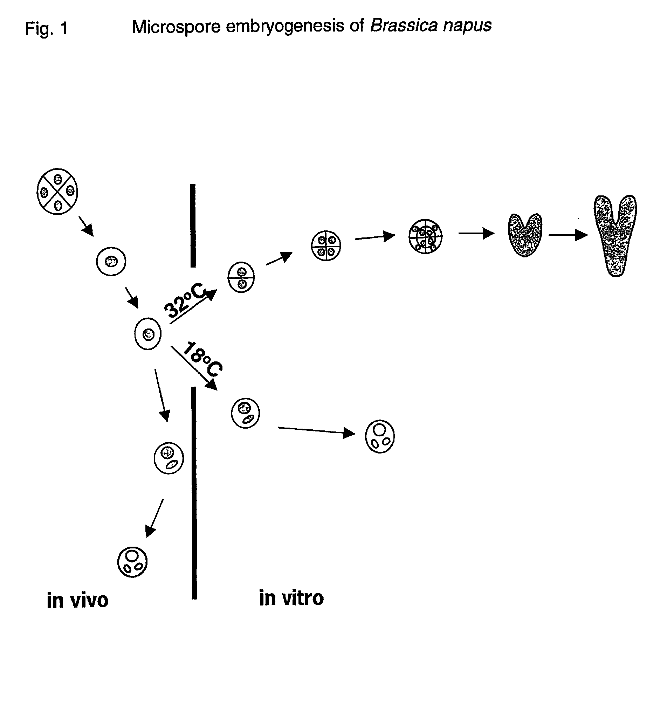

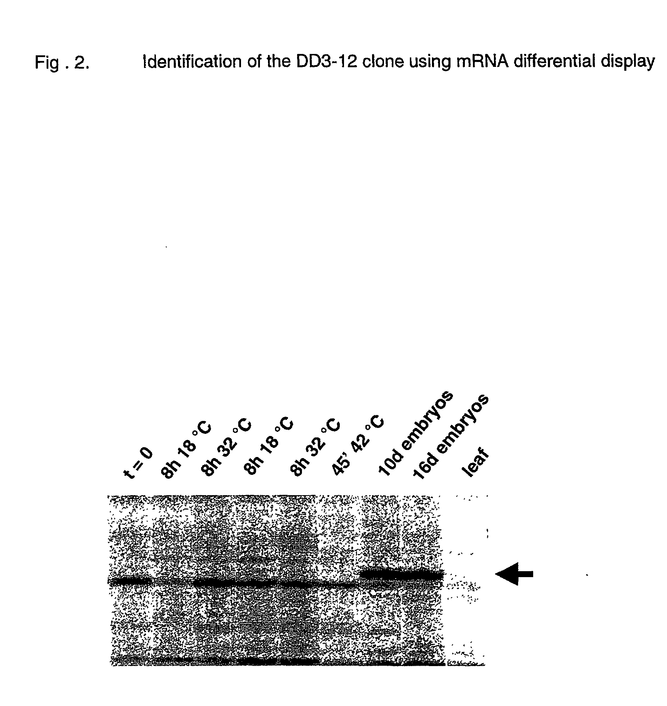

Isolated microspores of *Brassica napus* cv. Topas at a stage around the first pollen mitosis were cultured either at 32°C or at 18°C. The higher culture temperature leads to the formation of embryos and the lower culture temperature leads to pollen maturation (Fig. 1). Samples were collected at various days after initiation of the cultures and total RNA was prepared according to the procedure described in materials and methods. mRNA differential display RT-PCR (DDRT-PCR, Liang and Pardee, 1992) was used to isolate cDNA clones which appeared specifically under embryogenic conditions (32°C). The DDRT-PCR gel of Fig. 2 shows a PCR fragment (named *LLP1Bn*, indicated by an arrow) that was found in the samples of microspores cultured for 10 days (globular to heart shaped embryos) and 16 days (heart to torpedo stage embryos) at 32°C (embryogenic development), but not in samples of freshly isolated microspores (t=0), microspores cultured at 18°C (gametophytic development) or in leaf. This *LLP1Bn* PCR fragment was isolated from the gel and sequenced after re-amplification and cloning. Comparison with DNA sequences in NCBI GenBank revealed no significant sequence homology with known genes.

A cDNA library prepared from globular to heart staged embryos was screened in order to clone the full length cDNA of *LLP1Bn.* This has led to the identification of a full length *LLP1Bn* cDNA (otherwise known as DD3-12), as shown in SEQ ID No.1 (Fig. 3). Analysis of this putative protein using SignalP programs (http://www.cbs.dtu.dk/services/SignalP/) indicated that this protein has a 23 amino acid hydrophobic transit peptide. Such a signal peptide will be removed during the transfer from inside the cell to outside. It is therefore expected that the final product of this peptide has only 51 amino acids. Proteins with such characteristics are normally working as a ligand protein interacting with one or several receptor kinases in the membrane of surrounding cells for signal transduction between cells (Jennifer and Meyerowitz, 1999).

### Example 2. Expression of the LLP1Bn gene

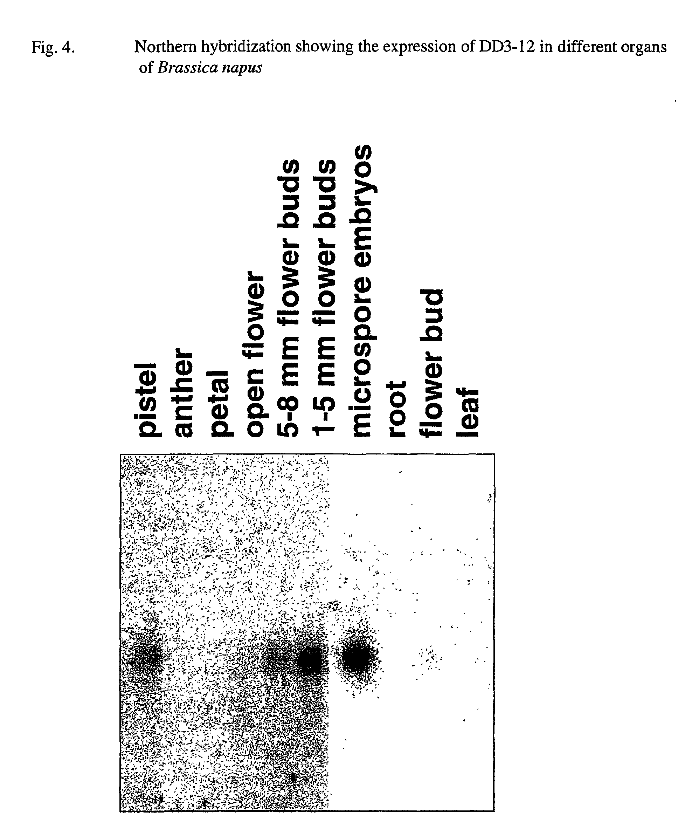

The expression pattern of LLP1Bn as determined by Northern blot analysis is shown in Fig. 4. A high level of transcript was found in microspore embryos of a 10 days 32°C culture (globular to heart shaped embryos). No signal was detected in root and leaf tissue, but a faint signal appeared in a mixture of flower buds of various developmental stages (Fig. 4). Separate sampling of RNA from younger buds (1-5 mm), older buds (5-8 mm) and open flowers revealed that the highest level of *LLP1Bn* transcript can be found in the youngest flower buds. Within a flower a clear signal was found in pistels, but not in anthers and petals.

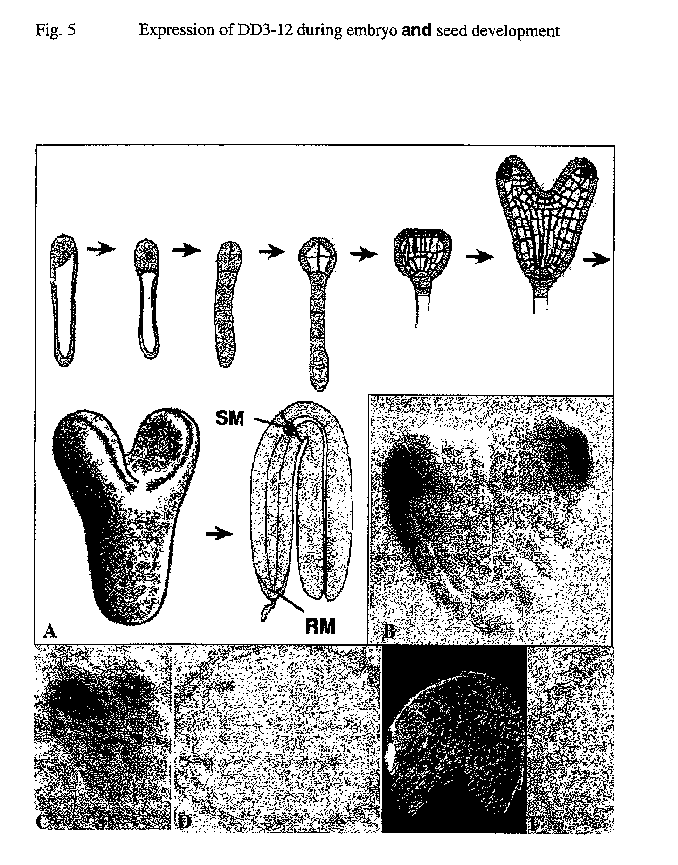

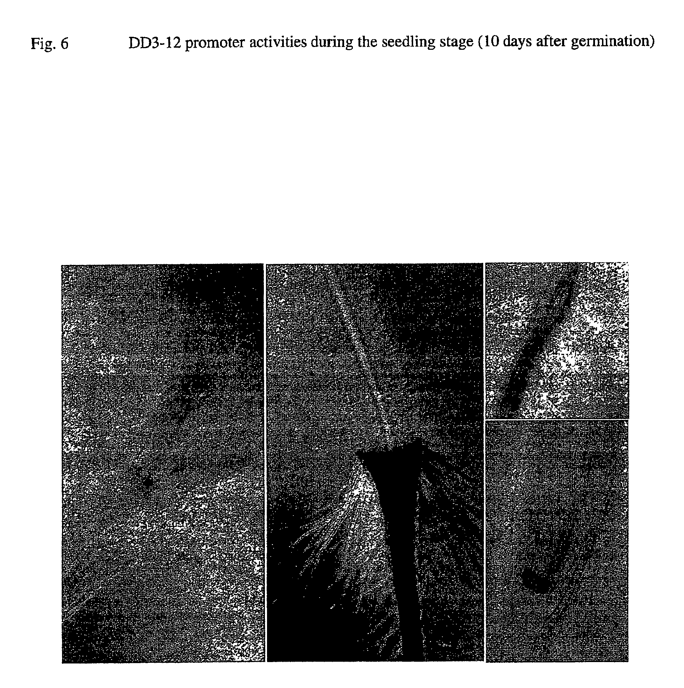

A *LLP1Bn promoter::*GUS fusion was constructed and transferred to *Arabidopsis* using a "floral dip" method (ref) to determine the expression pattern of LLP1Bn in a close relative of *Brassica - Arabidopsis thaliana.* Transgenic seedlings were selected on plates containing Kanamycin (Fig 6). The GUS signal was first detected in the upper part of the embryos at later globular stage. At the heart-shape stage the GUS expression is restricted to the top but slightly close to the abaxial side of the cotyledons. Further development of the embryo led to the change of the expression of LLP1Bn to a narrow tier of cells at the edge of the cotyledon (see Fig. 5). At the cotyledon stage the LLP1Bn expression was localized to a ring-shaped region at the base of each cotyledon, but not in the embryo including the root and apical meristems.

Interestingly during seed maturation, the expression of the LLP1Bn was seen in the remnant of the endosperm, a single layer of cells located between the testa and the embryo. The GUS expression was continued till the first few days of seed germination.

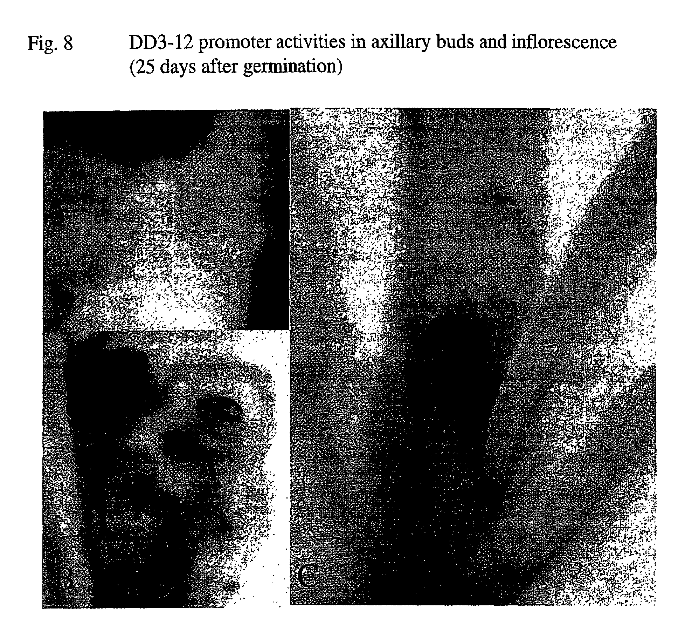

During post-embryonic development, the expression of LLP1Bn is restricted to axillary buds, flower buds and mature roots, not in leaf, flower, or vegetative meristems. In *Arabidopsis* each axillary bud will normally form one new inflorescence which has 2-5 cauline leaves and indefinite number of flowers. As soon as flower starts to form, no cauline leaf will be produced. Generally, only one inflorescence is produced from each axillary bud. In the axillary buds, the expression is restricted to leaf primordia and moved quickly to the abaxial side of the peteols when leafs are expanding (Fig. 8C). In the flower buds, the LLP1Bn expression was first seen in the stage 3 flower buds at a periphery of the flower primordia indicating the positions where sepals are forming. In a stage 5 flower, the LLP1Bn is no more expressed in the sepals which has already formed, it is in a region between sepal and carpel primordia, where petals and stamens are going to be formed. In a stage 7 flower, when stamens are forming the LLP1Bn expression is seen only at they top of the carpel where stigma is forming. The expression of LLP1Bn was switched off completely before the flower opens.

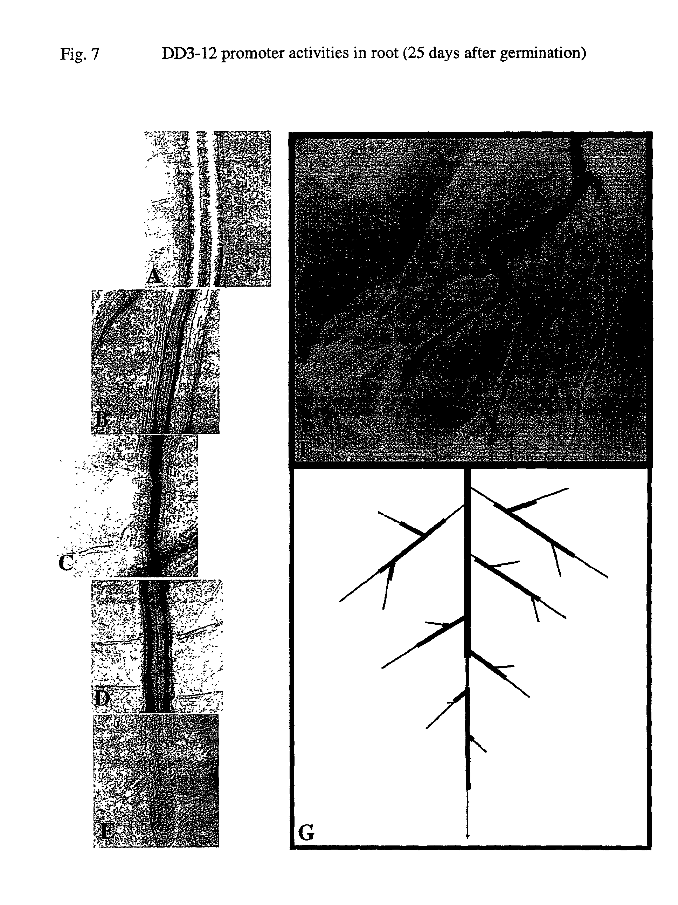

In roots, the expression of LLP1Bn started after root hairs are formed, 6-7 days after germination (Fig. 7). No expression can be seen in the hypocotyl and the expression margin between hypocotyl and root are very sharp. Within the root, LLP1Bn expression was excluded from the epidermal layer on which the root hairs will be formed. The LLP1Bn expression was gradually switched off in the cortex and the ground tissue to the vascular boundles, and later to the pericycle and then off completely when the root hair starts to degenerate. Apparently the LLP1Bn expression is associated with mature roots with well developed root hair. This is the region where root functions dominantly for nutrient intake from soil. No LLP1Bn expression was seen during lateral root induction, nor the old root which functions as a supporting and transporting organ.

### Example 3 The phenotype of LLP1Bn over-expression

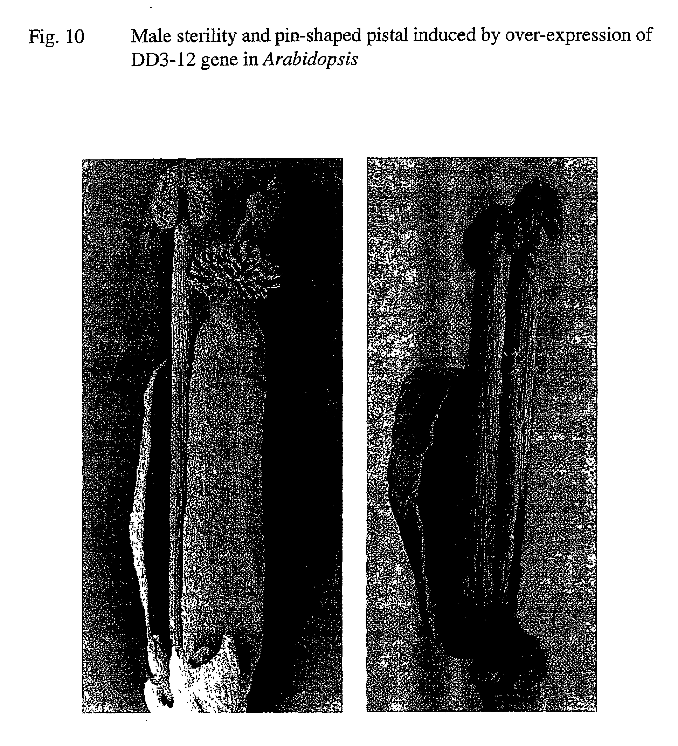

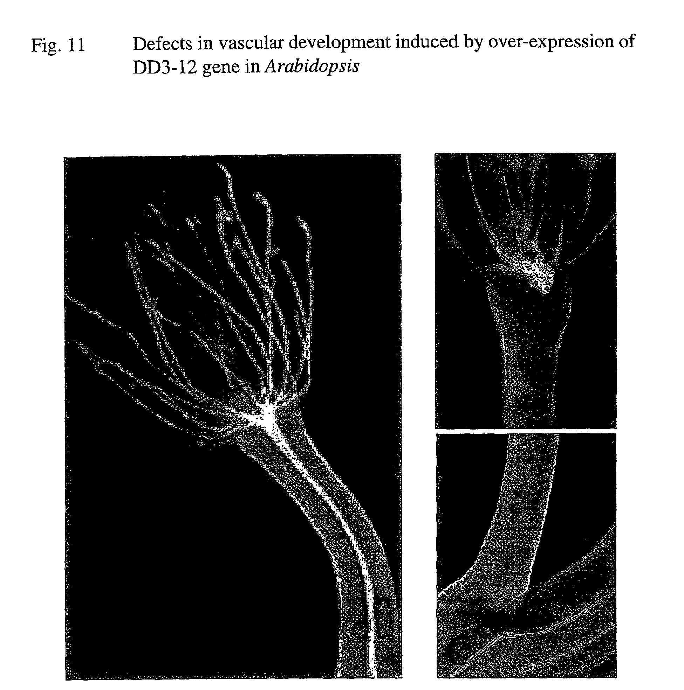

Doubled enhanced 35S promoter was used to drive the over-expression of full length of LLP1Bn gene (otherwise known as DD3-12) in Arabidopsis. The transformation was carried out using the floral dip method mentioned previously. Three independent transformants with almost identical phenotype were obtained from 2 transformation experiments. These plants are slow growing and late flowering, bolting only 45-50 days after seeds were planted instead of 20 days in the WT. A dramatic phenotype of these LLP1Bn over-expression plants is their changes in branching patterns (Fig 9). Instead of one branch was formed at each axillary buds, these plants normally have 2, 3, 4, 5, and even 7 inflorescence produced at the axillary position of cauline leaves. The formation of new inflorescence are gradual, starts with one branch and new ones are formed during the growth of the plants. These LLP1Bn over-expression plants are male sterile, no viable pollen can be produced in the flower. The anther also stays very small, in a triangle shape. Another change in the LLP1Bn over-expression plants is the formation of pin-shaped carpel in 80% of the flowers (Fig 10). These pin-shaped carpels are slender structure without formation of ovules inside. A stigma-like structure can be observed at the top of the carpel, indication that the expression of LLP1Bn may function as a signal cue for ovule induction, rather than the formation of the stigmatic tissue. Those 20% flowers with normal pistil are fertile if pollinated with pollen from wildtype plants. A careful cytological analysis has showed that the LLP1Bn ever-expression plants have defects in building up vascular strands, especially in flowers (Fig 11).

### Example 4. The identification of other LLPs in Arabidopsis

General BLAST or BLASTP searches through NCBI and Arabidopsis database using either LLP1Bn cDNA sequence or protein sequence showed no significant homology with any known cDNA or protein sequences. Our first attempt in searching existing protein databases SWISSPROT using BLASTP in NCBI and the TAIR (an Arabidopsis database) GenBanks have showed no significant matching sequences. However, based on the sequence alignment between LLP1Bn and LLP1At proteins we found that the C-terminals of these two proteins are highly conserved, which might be associated with the important function of these genes (Figures 12 & 13). We then used the C-terminal sequence to do the database search with a modification of several parameters (Fig 14). Instead of using protein-protein homologous search, we used the C-terminal peptide sequences to search all nucleotide sequences in NCBI and TAIR databases with 6-frame translation. This will allow us to access all the possible sequences in the databases. Such a search has led us to the identification of a group of proteins with highly conserved C-terminal boxes, thirteen of them are from Arabidopsis (Fig 15), one from cotton and one from soyabean. This is a boxmotif which has never been identified before. We termed it as LLP boxmotif. Interestingly, all these proteins are very small, ranging from 60 to 120 amino acids (see for examples figures 17-22). Additional members have been identified which belong to the LLP family using this search criteria (fig 23).

All of these proteins with a LLP boxmotif have an N-terminal signal peptide with 15 to 32 amino acids, as indicated by SignalP, analysis (http://www.cbs.dtu.dk/services/SignalP/). Such signal peptides control the entry of virtually all proteins to the secretory pathway to outside of the cells. The signal peptide will be cleaved off while the protein is translocated through the membrane. The common features of these signal peptides are a positively charged n-region, followed by a hydrophobic h-region and a neutral but polar c-region. A (-3,-1) rule states that the residue at the position -3 and -1 (relative to the cleavage site) must be small and neutral for cleavage to occur correctly (Nielsen et al, 1997).

Based on these three common features, it becomes apparent that LLP is a new class of protein. They may function as ligands to interact with receptor kinases in the neighboring cells for cell-cell communication.

Since LLP genes encode ligands that are able to interact with membrane bound receptor kinases in order to induce a signal transduction cascade, it is possible to make use of this interaction for other purposes. Redesigning a LLP ligand in such a way that a stable non-productive interaction occurs between it and its receptor will result in a competition between the modified and the wild-type ligand for receptor binding. Substituting certain amino acids in the receptor interaction domain will create a stable, non-functional ligand that will occupy the receptor binding sites, resulting in a dominant negative mutant phenotype where the signal transduction cascade is blocked. This can be used to alter plant architecture. The interacting domains of the ligand and receptor can also be used in a different context, by linking them to other proteins that normally would not interact. In this way new protein-protein interactions can be created in planta.

Several known proteins have certain similarities to the LLP proteins. These are CLAVATA3 protein from arabidopsis and ESR protein from maize. They are also small proteins with a signal peptide at their N-terminals. These proteins showed certain similarities with LLP in the LLP boxmotif as well, certainly the similarity is lower. The most distinct differences are the location of the LLP boxmotif. A somewhat alike box in CLAVATA3 and ESR proteins is located much further away from the C-terminal end than LLP.

As mentioned above, most LLP proteins shown here have the LLP boxmotif 0-3 amino acids away from their C-terminal ends. One animal protein, a putative RHO/RAC guanine nucleotide exchange factor (RHO/RAC GEF) isolated from mouse (accession No. P52734), also showed certain homology with LLP proteins in the LLP boxmotif. In this case, however, the assumed LLP boxmotif is much further away from the C-terminal, and even closer to the N-terminal (Pasteris, et al, 1995). This is the first time that the LLP sequence motif has been identified in any organism. In plants we find this motif generally associated with small extracellular ligand-like proteins.

### Example 5 : Finding novel LLP genes and peptides

Given the conserved nature of the LLP motifbox, the public sequence databases were searched for sequences or putative ORFs that encode proteins containing a K **R** X (V/I) (P/H) (S/T) **G** (P/S) (N/D) (P/H) (L/I) **H** (H/N) domain. Many Arabidopsis LLP box-containing ORFs were identified. Many of these were not yet annotated in the database. Extrapolating from the LLP box, the start and stop codons of the ORF were identified. The size of the predicted proteins encoded by these ORFs ranged from 100 to 250 amino acids, and all had a high probability of encoding a signal peptide at their N terminus. The following Arabidopsis ORFs belong to the LLP family based on the size of the predicted protein, the likelyhood of a amino terminal signal peptide, and the presence of the LLP box:
- Located on chromosome 1, on BAC clone F23o10 accession AC018364 (34983 until 34660) from *Arabidopsis thaliana*
- Located on chromosome 1, on BAC clone F2OP5 accession AC002062 (108432 until 108788) from *Arabidopsis thaliana*
- Located on chromosome 1, on BAC clone T1K7 accession AC013427 (8397 until 8759) from *Arabidopsis thaliana*
- Located on chromosome 2, on BAC clone F2I9 (section 4 of 255 of chromosome 2) accession AC006069 (55097 until 55786) from *Arabidopsis thaliana*
- Located on chromosome 1, on BAC clone T7A14 accession AC005322 (16956 until 17207) from *Arabidopsis thaliana*
- Located on chromosome 1, on BAC clone F12A21 accession AC008113 (31668 until 31928) from *Arabidopsis thaliana*
- Located on chromosome 1, on BAC clone F15H11 accession AC008148 (45836 until 46069) from *Arabidopsis thaliana*
- Located on chromosome 1, on BAC clone F27J15 accession AC016041 (90633 until 90932) from *Arabidopsis thaliana*
- Located on chromosome 1, on BAC clone F9N12 accession AC022355 (48056 until 48298) from *Arabidopsis thaliana*
- Located on chromosome 1, on BAC clone F2P9 accession AC016662 (57033 until 57356) from *Arabidopsis thaliana*

Additionally, a number of expressed sequence tags (EST's) and genes with previously unknown functions that were found in the database, belong to the LLP family, based on the criteria mentioned above. These include:
- thaliana, Columbia Col-0, rosette-2 Arabidopsis thaliana cDNA clone 701546165, mRNA sequence
   gi |5845463 |gb | AI998558.1| AI998558[5845463]
- thaliana, Ohio State clone set Arabidopsis thaliana cDNA clone 701496429, mRNA sequence gi |5840376| gb |AI993471.1| AI993471[5840376]
- Zea mays endosperm cDNA library from Schmidt lab cDNA, mRNA sequence gi |4887284| gb | AI677383.1| AI677383[4887284]
- Z.mays mRNA for ESRa1 protein gi |2340960| emb | X98495.1 | ZMRESRA1[2340960]
- Z.mays mRNA for ESR2c1 protein gi |2340958| emb |X98498.1|ZMRESR2C1[2340958]
- Z.mays mRNA for ESR1c1 protein gi |2340956| emb |X98496.1| ZMRESR1C1[2340956]
- Z.mays ESR3g2 gene, clone L42a4 gi |2340954 emb | X99970.1 |ZMESR3G2[2340954]
- Z.mays ESR2g2 gene, clone L42a14 gi | 2340952 | emb | X99969.1 | ZMESR2G2[2340952]
- Z.mays ESR1g2 gene, clone L42a6 gi | 2340950 | emb | X99968.1 | ZMESR1G2[2340950]
- Z.mays ESR2g1 gene gi |2340948| emb | X98499.1 | ZMDESR2G1[2340948]
- Z.mays ESR1g1 gene gi |2340946| emb |X98497.1| ZMDESR1G1[2340946]
- Rice cDNA from immature leaf including apical meristem Oryza sativa cDNA clone E51222_2Z, mRNA
   sequencegi |3763791| dbj | AU030543.1| AU030543[3763791]
- Cotton Six-day Cotton fiber Gossypium hirsutum cDNA 5', mRNA sequence
- gi |6462118| gb | AW187682.1| |AW187682[6462118]
- Soybean Glycine max cDNA clone GENOME SYSTEMS CLONE ID: Gm-c1016-2901 5', mRNA sequence gi |6094825| gb | AW119439.1| AW119439[6094825]

The criteria used to identify these LLP proteins can be used to recognise new members of the LLP family as they appear in public databases.

### Experimental procedures

### Plant material and microspore culture

The cultivation of the doubled haploid *Brassica napus* L. cv. Topas plants and the isolation of microspore and pollen grains was performed as described. Plants were raised all year round in a phytotron room at 18°C with a 16 h photoperiod. Microspores and pollen were isolated by disrupting flower buds with a pestle in NLN medium (Lichter, 1982) containing 13% (w/v) sucrose (NLN13). Late unicellular microspore and early bicellular pollen were cultured in NLN13 medium at a density of 40,000 cells/ml, either at 18°C (gametophytic development) or at 32°C (embryogenic development).

### Nucleic acid isolation

Total RNA from microspore cultured at 18°C (8 h), 32°C (8 h) or 41°C (45 min) was isolated using an extraction buffer containing a 1:1 mixture of phenol and 0.1 M LiCl, 10 mM EDTA, 1% SDS, 0.1 M Tris-HCl (pH 8.0). One ml of hot (60°C) extraction buffer was added to the microspore pellet (approx. 10⁶ microspore) and the homogenate was rigorously vortexed in the presence of glass beads. After centrifugation the aqueous phase was extracted with an equal volume of chloroform and the RNA was precipitated at -20°C by the addition of 1/3 vol of 8 M LiCl. The pellet was washed with 70% ethanol, dried and dissolved in diethylpyrocarbonate (DEPC)-treated water. All other total RNA samples were obtained by grinding the plant material in liquid nitrogen with a mortar and pestle, and subsequent extraction of the fine powder using TRIZOL reagent (Gibco-BRL). Genomic DNA was isolated from leaf tissue according to Fulton *et al*., 1995, and digested with the specified restriction enzymes according to procedures suggested by the manufacturer (Gibco-BRL).

### Differential display

Differential display (Liang and Pardee, 1992)of mRNA was performed using RNAmap Kit B (GenHunter, USA) according to the manufacturer's recommendation. Total RNA from freshly isolated microspore, microspore cultured at 18°C (8 h), 32°C (8 h, 10 d, 16 d) or 41°C (45 min), and leaf tissue of *B. napus* was isolated as described above and DNAse I treated using the MessageClean Kit (GenHunter). Differential display was carried out on two independent 8 h cultures of 18°C and 32°C. A real heat-shock was given by treatment of microspore at 41°C, a condition that does not lead to embryogenesis in microspore of this developmental stage. DNAse-free total RNA samples (0.2 µg) were used for the first strand cDNA synthesis. Four T₁₂MN anchor primers (where M is degenerate A, C, G and N is either A, C, G or T) were used in four reverse transcription (RT) reactions. PCR amplification of one-tenth of the first-strand synthesis cDNA products was done in the presence of [α-³³P]dATP. Five decamers (AP₆ to AP₁₀) were used in combination with the respective T₁₂MN. All PCR steps were performed using the Perkin-Elmer GenAmp 9600 system and AmpliTaq polymerase from Perkin-Elmer. The amplified [α-³³P]dATP labeled cDNAs were resolved on 6% denaturating polyacrylamide gels containing 7 M urea. After drying the gels on Whatman 3MM paper and autoradiographic detection of bands, differentially expressed cDNAs were excised and eluted according to the manufacturer's instructions. cDNAs were then re-amplified using the same PCR conditions and primers as before. PCR products were analysed on a 1.2% agarose gel and cDNA fragments of interest were eluted and cloned into the pGEM-T vector (Promega). To confirm the differential display pattern the cloned cDNAs were used as probes for RNA blot hybridizations.

### DNA and RNA gel blot analyses

DNA fragments were separated in 1% agarose and transferred overnight onto Hybond-N⁺ (Amersham) by capillary blotting with 20xSSC. For RNA gel blot analysis, 10 µg of total RNA was denatured with glyoxal prior to electrophoresis and blotting onto Hybond-N⁺ membrane. After ultraviolet cross-linking the membranes were hybridized with a [³²P] random-primer-labelled probe of the DD-clone of LLP1Bn. Membranes were hybridized overnight at 65°C in 10% dextran sulphate, 1% SDS, 1 M NaCl, 50 mM Tris-HCl (pH 7.5) and washed first 30 min twice at moderate stringency (65°C, 2xSSC, 1%SDS), followed by two 30 min high-stringency washes (65°C, 0.2xSSC, 0.5%SDS).

### cDNA library construction and screening

Poly(A)⁺ RNA was isolated from total RNA of globular to heart stage *B. napus* microspore embryos using Poly(A) Quik columns (Stratagene). Five µg poly(A)⁺ RNA was used as starting material for the construction of an Uni-ZAP XR cDNA library (Stratagene). Approximately 10⁶ plaques were screened under high-stringency conditions with the cDNA as isolated by DDRT-PCR (Fig. 3 ). One positive clone was isolated, purified and sequenced (Fig. 3).

### Isolation of promoter sequence

The Universal Genome Walker Kit (Clonetech) was used to isolate genomic DNA fragments lying upstream of the *LLP1Bn* ATG start codon. Pools of uncloned, adaptor-ligated *Brassica napus* cv Topas genomic DNA fragments were constructed and used to isolate *LLP1Bn* genomic sequences by nested PCR. The primary PCR made use of the outer adapter primer (AP1) supplied by the manufacturer and a *LLP1Bn* specific primer with the sequence: 5'-CCATTCTTCATCAGCAAACTCCGAAATGA-3' The nested PCR made use of the nested adapter primer (AP2) supplied by the manufacturer and a *LLP1Bn* specific primer with the sequence: 5'-CAGAAAAGAGGAAGCCAATATCAAACTC-3' The primary PCR mixture was then diluted 1:50 and used as template for nested PCR. Both the primary and nested PCRs were performed as recommended by the manufacturer. The nested PCR products were cloned into the pGEMT vector (Promega) and sequenced. PCR products corresponding to the 5' untranslated genomic region of *LLP1Bn* cDNA were identified (Fig 16).

### Plasmid construction for plant transformation

The construction of a plasmid vector containing the *LLP1Bn* cDNA under control of the double cauliflower mosaic virus 35S promoter withd AMV translational enhancer was as follows. The complete coding region of *LLP1Bn* was already cloned into the GST fusion vector pGEX4T-2 (Amersham Pharmacia Biotech). This plasmid was cut with the restriction enzymes BamHI and XhoI. The 231bp LLP1Bn fragment was isolated and ligated into the vector pGD121 (containing a double 35S promotor with AMV enhancer and a pBINplus backbone), already cut with the restriction enzymes BamHI and XhoI. This construct was confirmed by sequencing, and transformed to *A.tumefaciëns* C58PMP90.

### Promoter-GUS construct

The promotor LLPIBn-GUS was made as follows; a 1060bp LLP1Bn promotor fragment (obtained by genome walking) cloned in pGEM-T (PROMEGA) was used for this construction. This construct was used in a PCR with the primers: P312-1 5'-CGCTCTAGAGTTCTATCTTTGTCAAAAAAAA-3' anneals on the promotor, just before the ATG. P312-2 5'- ATATAAGCTTACTATAGGGCACGCGT-3' anneals on the genome walker adaptor.
As proofreading polymerase Pfu (STRATAGENE) was used. PCR protocol: 45 seconds at 94°C, 60 seconds at 40°C, 4.5 minutes at 72°C [cycle repeated twice] followed by a 45 seconds at 94°C, 60 seconds at 54°C, 4.5 minutes at 72°C [cycles repeated 18 times] followed by 3 minutes at 72°C and a 4°C hold.
The obtained fragment was cut with the restriction enzymes HindIII and XbaI, and ligated into the pRAP2T/GUS vector (containing GUS intron and the NOS terminator and a pUC vector as backbone) that was already digested with HinDIII and XbaI. This constuct was digested with PacI and AscI and a fragment containing the LLP1Bn promotor, GUS-intron and the NOS terminator was isolated and ligated into pBINplus, digested with PacI and AscI. The obtained vector was confirmed by sequencing and transformed to *A.tumefaciëns* C58PMP90.

### Plant transformation

*Arabidopsis thaliana* ecotype C24 was used as the recipient in transformation experiments. Plants were transformed using the flower dip method described in Clough and Bent (1998).

### Cryo-electron microscopy

Plant materials were glued to a copper stub using conductive carbon glue and immediately frozen in liquid nitrogen. The sample was then transfered to a low temperature field emission scanning electron microscope (LT-FESEM, JEOL JSM 6300F) equipped with an Oxford cryochamber. After a light coating with argon gas the samples were observed and pictures were taken with a digital camera.

**Table 1.**

| Examples of genes that regulating plant development | | | |
|---|---|---|---|
| **Gene function** | **Name** | **Protein identity** | **References** |
| **Cotyledon identity** | LEC1 | CCAAT box transcription factor | Lotan et al, 1998 |
| | CUC2 | putative transcription factor | Aida et al., 1997 |
| **Floral organ identity** | AG | MADS transcription factor | Yanofsky et al, 1990 |
| | AP1 | transcription factor | Mandel et al, 1992 |
| | AP2 | transcription factor | Jufuku et al, 1994 |
| | LFY | transcription factor | Weigel et al, 1992 |
| Shoot meristem identity | STM | KNOTTED-like transcription factor | Long et al, 1996 |
| | CLV1 | receptor kinase | Clark, et al, 1997 |
| | CLV2 | receptor kinase | Jeong et al, 1999 |
| | CLV3 | ligand protein | Fletcher and Meyerowitz, 1999 |
| Abaxial-adaxial cell fate | FIL | putative YABBY transcription factor | Sawa et al, 1999 |
| Vascular development | MP | auxin-responsive transcription factor | Hardtke and Berleth, 1998 |
| Signal transduction | BRI | receptor kinase | Li and Chory, 1997 |

### References

Aida, M., Ishida, T., Fukaki, H., Fujisawa, H and Tasaka, M. (1997) Genes involved in organ separation in *Arabidopsis:* an analysis of the cup-shaped cotyledon mutant. Plant Cell 9:841-857.
Chang, C. and Meyerowitz, EM (1995) The ethylene hormone response in Arabidopsis ― an eurokaryotic 2-component signalling system. Proc. Natl. Acad. Sci. USA 92: 4129-4133.
Citovsky, V and Zambryski, P. (1991) How do plant virus nucleic acids move through intercellular connections. BioAssays 13: 373-379.
Clark, S.E., Williams, R.W., Meyerowitz, E.M. (1997) The CLAVATA1 gene encodes a putative receptor kinase that contols shoot and floral meristem size in Arabidopsis. Cell, 89:575-585.
Clough, S.J. and Bent, A.F. (1998) Floral dip: a simplified method for Agrobacterium-mediated transformation of Arabidopsis thaliana. Plant J. 16: 735-743.
Fletcher, J.C., Brand, U., Running, M.P., Simon, R. and Meyerowitz, E.M. (1999) Signaling of cell fate decision by CLAVATA3 in Arabidopsis shoot meristems. Science 283:1911-1914.
Jinn, T.-L., Stone, J.M. and Walker, J.C. (2000) HAESA, an Arabidopsis leucine-rich repeat receptor kinase, controls floral organ abscission. Genes and Development 14:108-117.
Kakimoto, T. (1996) Cki1, a histidine kinase homolog implicated in cytokinin signal-transduction. Science, 274:982-985.
Ku, T., Mitsukawa, N., Oosumi, T., Matsuura, R., Whittier, R.F. and Komeda, Y. (1996), The Arabidopsis ERECTA gene encodes a putative receptor protein kniase with extracellular leucine-rich repeats. Plant Cell, 8:735-746.
Liang, P., and Pardee, A.B. (1992) Differential display of eukaryotic messenger RNA by means of the polymerase chain reaction. Science 257: 967-971.
Lichter (1982). Induction of haploid plants from isolated pollen of Brassicia napus. Z.Pflanzen-Physiol. 105 : 427-434.
Lawton, MA, Yamamoto, RT, Hanks, SK, Lamb, CJ (1989) Molecular cloning of plant transcripts encoding protein kinase homologs. Proc. Natl. Acad. Sci. USA 86:3140-3144.
Lucas W.J., Bouche-Pillon, S., Jackson, D.P., Nguyen, L., Baker, L., Ding, B. and Hake, S. (1995) Selective trafficking of Knotted1 homeodomain protein and its mRNA through plasmodesmata. Science 270:1980-1983.
Hake S. and Char, B. (1997) Cell-cell interactions during plant development. Genes and Development 11:1087-1097.
Sawa, S., Watanabe, K., Goto, K., Kanaya, E., Morita, E.H., Okada, K. (1999) FILAMENTOUS FLOWER, a meristem and organ identity gene of Arabidopsis encodes a protein with a zinc finger and HMG-related domains. Genes Dev., 13:1079-1088.
Van den Berg, C.V., Willemsen, V., Hage, W., Weisbeek, P. and Scheres B. (1995) Cell fate in the Arabidopsis root meristem determined by directional signalling. Nature 378:62-65.
Fletcher, J.C. and Meyerowitz, E.M. (2000) Cell signalling within the shoot meristem. Current Opinion in Plant Biology, 3:23-30.
Fulton, T.M., Chunwongse and Tanksley (1995) Microprep protocol for extraction of DNA from tomato and other herbaceous plants. Plant Molecular Biology Reporter, 13(3): 207-209.
Li, J.M. and Chory, J. (1997) A putative leucine-rich repeat receptor kinase involved in brassinosteroid signal transduction. Cell, 90: 929-938.

### Figure Legends

- Fig. 1.: A diagram showing the microspore embryogenesis system we used to identify genes involved in embryogenesis. Late unicellular microspores and early bi-cellular pollen isolated from B. *napus* 'Topas' developed into embryos when cultured at 32°C, while the same population of cells continued gamethophytic developement into mature pollen when cultured at 18°C. Embryo or pollen materials can be harvested at different stages from these two conditions for RNA isolation.
- Fig. 2.: Identification of the LLP1 clone using differential display technique. A portion of a differential display gel showing the presence of LLP1 cDNA in 10-day and 16-day microspore-derived embryos. The RNA samples were prepared from the following materials:
- freshly isolated microspores (t=0);
- microspores cultured for 8 hr at 18°C (8h 18°C);
- microspores cultured for 8 hr at 32°C (8h 32°C);
- same as lane 2, but a different RNA isolation;
- same as lane 3, but a different RNA isolation;
- microspores that were heat-shock treated at 42°C for 45 minutes (no embryos will be produced from such treatment, 45' 42°C);
- microspore-derived embryos isolated from 10 days-old culture (10d embryos);
- microspore-derived embryos isolated from 16 days-old culture (16d embryos);
- leaves (leaf).
Note that, among these 9 RNA samples, the LLP1 signal (indicated by an arrow) was only seen in the lanes where the RNAs were isolated from microspore-derived embryos after 10 and 16 days culture.
- Fig. 3: The cDNA and protein sequence of LLP1. The top strand shows the cDNA isolated from a cDNA library of *Brassica napus* "Topas", and the bottom strand shows the fragment isolated originally by DD-PCR. The coding region together with the amino acid sequence was underlined, the signal peptide is double underlined, and the LLP boxmotif boxed.
- Fig. 4: Northern blot hybridization showing the expression of LLP1 gene in different organs and tissues of *Brassica napus* "Topas". Total RNAs were isolated from tissues marked above the gel and hybridised with labelled LLP1 fragment from DD-PCR.
- Fig. 5: Expression of LLP1 gene during embryo and seed development in *Arabidopsis thaliana.*
A. LLP1 Diagrammatic drawing shows the expression pattern of the gene, as revealed by LLP1 promoter::GUS fusion.
B-F. GUS staining of a late globular stage (B) and a heart-shape stage (C), cotyledonary stage (D) embryos and mature seed (E) and seed coat after germination (F). These results were obtained by GUS staining of transgenic plants carrying LLP1 promoter::GUS fusion construct. The LLP1 gene is expressed firstly in a late globular embryo (as marked in red) and restricted to the top of the cotyledons (as showed in C) and later to the edge of the cotyledon at the torpedo stage. In cotyledonary embryos the expression is restricted to the base of the cotyledon, not in the apical meristem. The expression was switched off in the embryo thereafter. In mature and germinating seeds, the expression is restricted to the remaining endosperm (also called aleurone layer, E and F).
- Fig. 6: LLP1 promoter activity in the seedling stage (10 days after germination).
A. Shoot apex;
B. Hypocotyl and root;
C. Main root and lateral root;
The LLP1 gene is not expressed in young seedling within 5 days of germination. In 10-day old seedlings, the LLP1 gene starts to express in the axillary buds (A) and roots with well-established root hairs (B). Such expression was excluded from the epidermal layer and the roothair. Note that no expression was seen in the hypocotyl (B) and the newly formed side roots (C).
- Fig. 7: The LLP1 promoter activities in roots. The staining was carried out in seedlings 25 days after seed germination.
A-E. A series pictures taken from one root at different positions. The expression of the LLP1 gene is absence in the root-tip (E), highest in the root hair region (D) and gradually restricted to the vascular bundles (C and D) and disappeared in mature roots (A).
F. A diagrammatic representation of the LLP1 expression in the root system, as indicated in red.
G. Transverse section through the upper part of the root hair region indicating that the expression is mainly in the vascular system.
- Fig. 8: LLP1 promoter activities in the axillary buds and the inflorescence (25 days after seed germination).
A. Longitudinal section through a young axillary bud revealing the expression of the LLP1 gene is only in the periphery of the apical meristem.
B. A developing axillary bud showing the promoter activity in the leaf primordia but not in the central meristem.
C. LLP1 gene is not expressed in mature leaves and stems.
D. Young flower buds showing the LLP1 expression in the region between sepal and carpel primordia in young flower buds and then in the stigmatic cells. These cells form a two-lip structure at the beginning and a ring at the later stage.
- Fig. 9: Changes of branching patterns in *Arabidopsis thaliana* "C24" induced by the over-expression of the LLP1 gene under the control of 35S promoter.
A. Electron microscopy photography showing a wild type stem with one shoot normally formed from each axillary bud.
B. Electron microscopy photography showing 3 inflorescences were formed from one axillary bud.
C. At the later stage of plant development, more than 6 shoots could be seen from one axillary bud.
- Fig. 10: Male sterility and pin-shaped pistil induced by the over-expression of LLP1 gene in *Arabidopsis thaliana* "C24".
A. Wild type flower observed by electron microscopy.
B. Flower from a 35S:LLP1 transgenic plant showing the anther without viable pollen grains and pin-shaped pistil. No ovule was formed within such a pin-shaped pistil. Some flower organs have been removed when the electron microscopy materials were prepared.
- Fig. 11: Defects in vascular development induced by over-expression of LLP1 gene in Arabidopsis *thaliana* "C24".
A. A wild type flower showing normal xylem formation.
B, C. Flower from LLP1 over-expression plants showing the failure of xylem connection between flower and main stem.

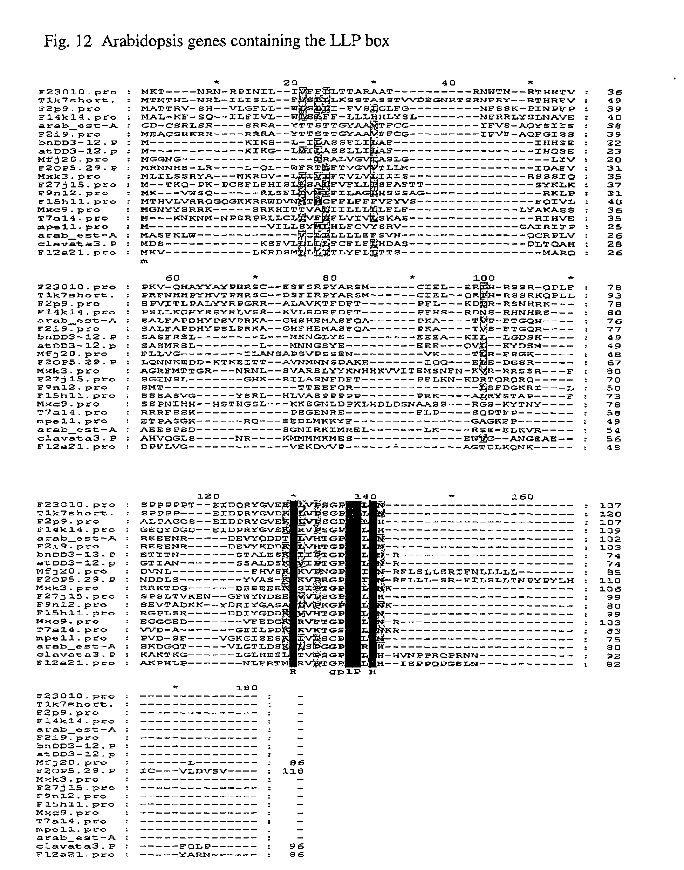

- Fig 12.: Arabidopsis genes containing the LLP boxmotif

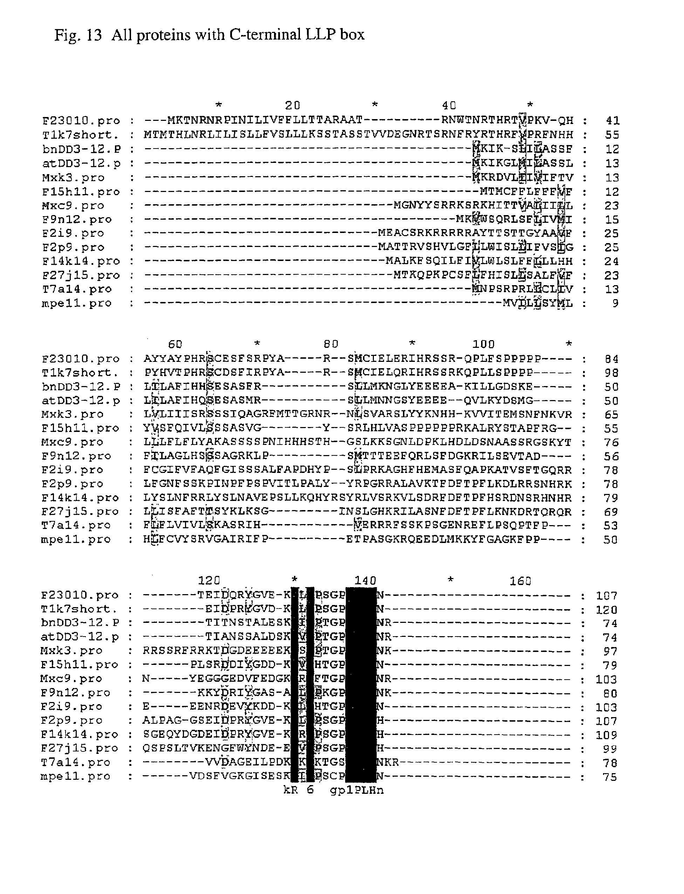

- Fig 13.: All proteins with C-terminal LLP boxmotif

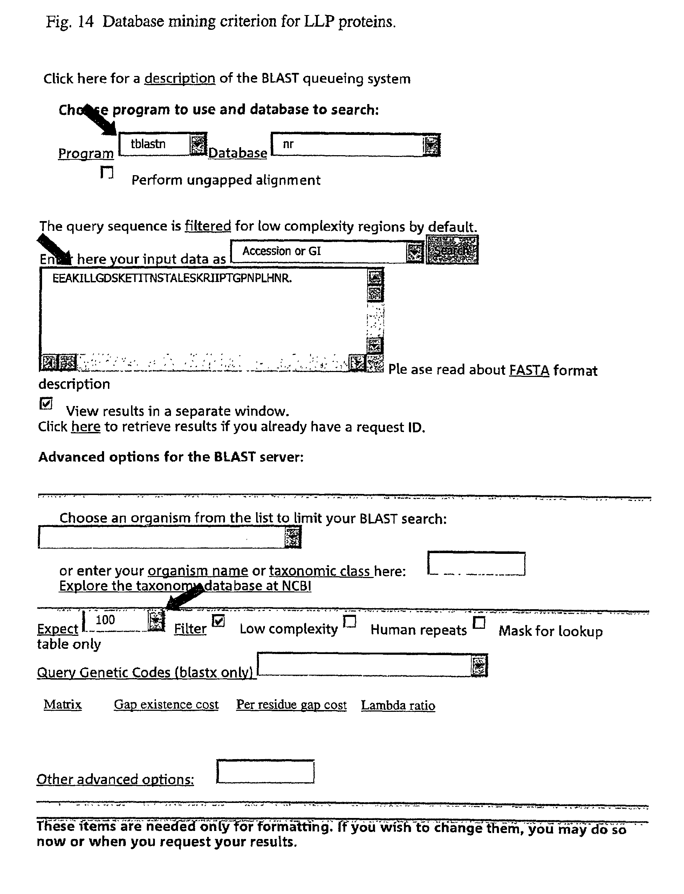

- Fig 14.: Database mining criterion for LLP proteins

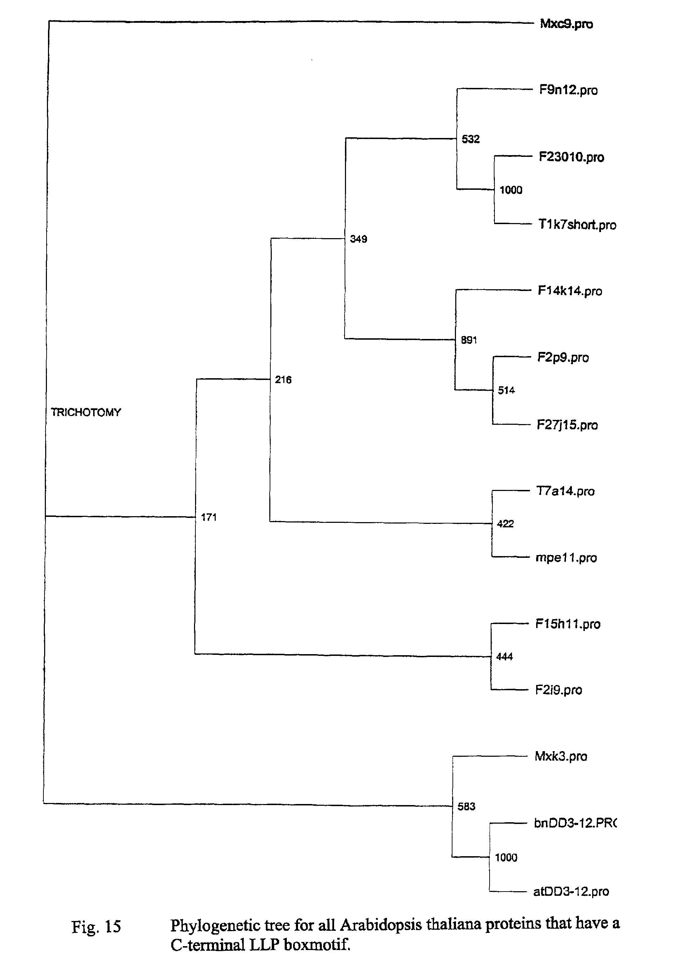

- Fig 15.: Phylogenetic tree for all Arabidopsis thaliana proteins that have a C-terminal LLP boxmotif.
- Fig 16.: The promoter sequence of BnLLP1

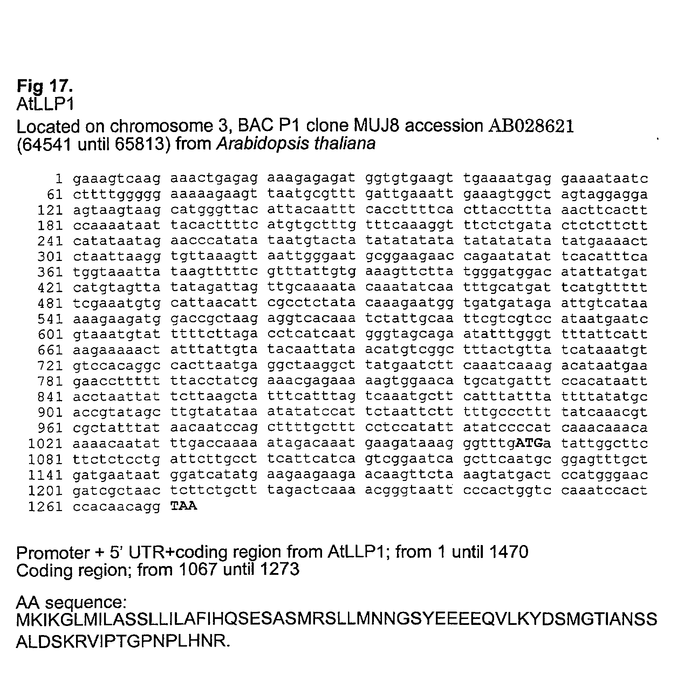

- Fig 17.: AtLLP1: Located on chromosome 3, BAC P1 clone MUJ8 accession B028621 (64541 until 65813) from *Arabidopsis thaliana*

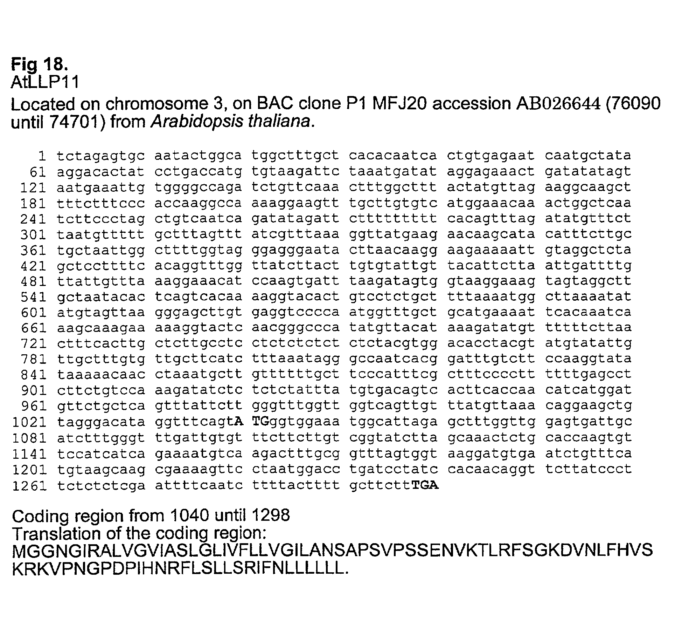

- Fig. 18.: AtLLP11: Located on chromosome 3, on BAC clone P1 MFJ20 accession AB026644 (76090 until 74701) from *Arabidopsis thaliana.*

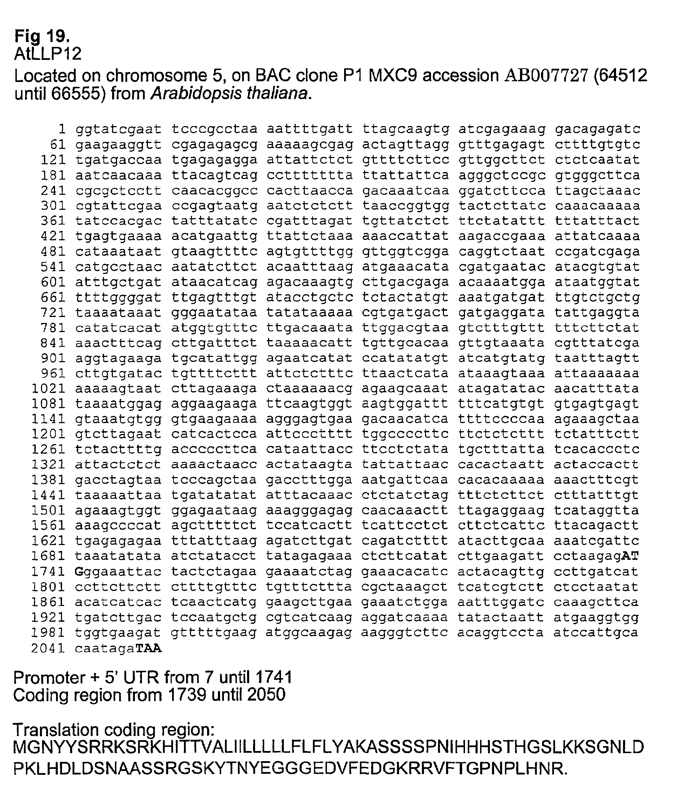

- Fig. 19.: AtLLP12: Located on chromosome 5, on BAC clone P1 MXC9 accession B007727 (64512 until 66555) from *Arabidopsis thaliana.*

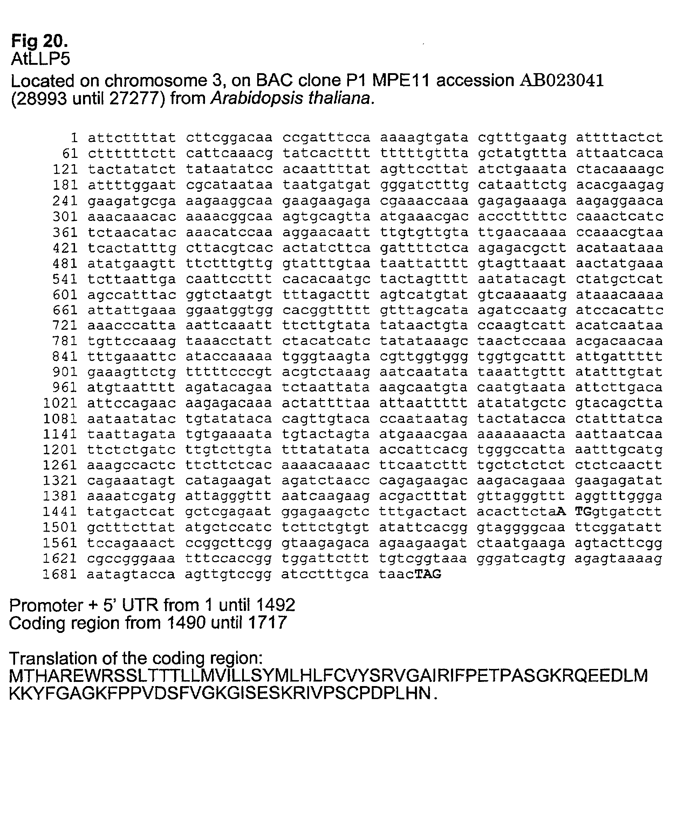

- Fig. 20.: AtLLP5: Located on chromosome 3, on BAC clone P1 MPE11 accession AB023041 (28993 until 27277) from *Arabidopsis thaliana.*

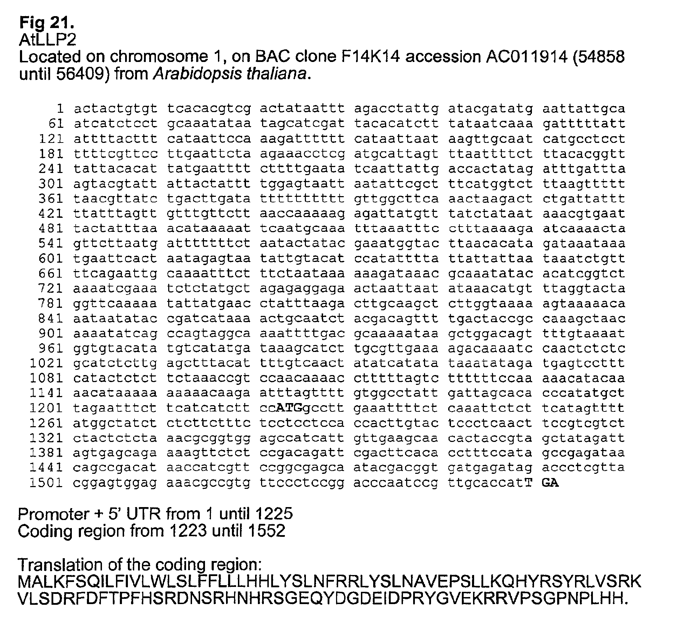

- Fig 21.: AtLLP2: Located on chromosome 1, on BAC clone F14K14 accession AC011914 (54858 until 56409) from *Arabidopsis thaliana.*

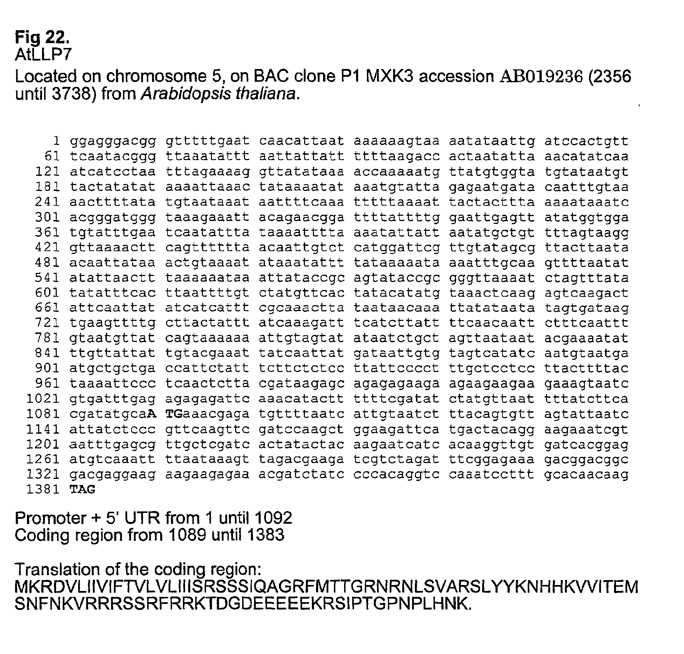

- Fig. 22.: AtLLP7: Located on chromosome 5, on BAC clone P1 MXK3 accession AB019236 (2356 until 3738) from *Arabidopsis thaliana.*

## Claims

1. A method for modulating plant phenotype comprising providing a plant with ligand-like protein (LLP) or a functional fragment thereof, said protein or fragment at least comprising a box comprising an amino acid motif XRXXXXGXXXXHX (LLP box).

2. A method according to claim 1 wherein said box comprises an amino acid sequence K R X (V/I) (P/H) (S/T) G (P/S) (N/D) (P/H) (L/I) H (H/N) or a motif at least 80% homologous therewith.

3. A method according to claim 1 or 2 wherein the C-terminus of said box is located at the most from about 10 amino acids away from the C-terminus of said ligand-like protein or functional fragment thereof.

4. A method according to anyone of claims 1 to 3 wherein said ligand-like protein (LLP) or functional fragment thereof comprises an N-terminal signal peptide.

5. A plant or plant material provided with a proteinaceous substance comprising a box comprising an amino acid motif XRXXXXGXXXXHX.

6. A plant or plant material according to claim 5 wherein said box comprises an amino acid sequence K R X (V/I) (P/H) (S/T) G (P/S) (N/D) (P/H) (L/I) H (H/N) or a motif at least 80% homologous therewith.

7. A plant or plant material according to claim 5 or 6 wherein the C-terminus of said box is located at the most from about 10 amino acids away from the C-terminus of said proteinaceous substance.

8. A plant or plant material according to anyone of claims 5 to 7 wherein said substance comprises an N-terminal signal peptide.

9. A plant or plant material according to anyone of claims 5 to 8 wherein said substance comprises at least about 50 amino acids.

10. A plant or plant material according to anyone of claims 5 to 9 wherein said substance comprises at the most about 250 amino acids.

11. A recombinant nucleic acid provided with a nucleic acid encoding a ligand-like protein or functional fragment thereof at least comprising a box comprising an amino acid motif XRXXXXGXXXXHX, or a nucleic acid hybridising therewith.

12. A recombinant nucleic acid according to claim 11 functionally linked with a promoter.

13. A recombinant nucleic acid comprising a promotor sequence or functional fragment thereof as provided in figure 16 or a nucleic acid functionally equivalent thereto.

14. A recombinant nucleic acid or functional fragment thereof according to claim 11, which is operably linked to a promoter or functional fragment thereof according to claim 13.

15. A vector comprising a nucleic acid according to anyone of claims 11 to 14..

16. A host cell comprising a nucleic acid according to anyone of claims 11 to 14 or a vector according to claim 15.

17. A plant or plant material comprising a cell according to claim 16.

18. A proteinaceous substance encoded by a nucleic acid according to anyone of claims 11 to 14.

19. A method for modulating plant phenotype comprising providing a plant or plant material with a nucleic acid according to anyone of claims 11 to 14, a vector according to claim 15 or a proteinaceous substance according to claim 18.

20. A plant comprising a modulated phenotype obtainable by a method according to claim 19.
